(19) Europäisches Patentamt European Patent Office Office européen des brevets

(11) **EP 4 224 189 B1**

(12) **EUROPÄISCHE PATENTSCHRIFT**

(45) Veröffentlichungstag und Bekanntmachung des Hinweises auf die Patenterteilung:
**02.07.2025 Patentblatt 2025/27**

(21) Anmeldenummer: **22154982.7**

(22) Anmeldetag: **03.02.2022**

(51) Internationale Patentklassifikation (IPC):
*G01R 33/28* (2006.01)    *G01R 33/341* (2006.01)
*G01R 33/3415* (2006.01)    *G01R 33/36* (2006.01)
*G01R 33/3875* (2006.01)

(52) Gemeinsame Patentklassifikation (CPC):
**G01R 33/285; G01R 33/3415; G01R 33/3635; G01R 33/3875;** G01R 33/34084

(54) **LAGEBESTIMMUNG EINER VORRICHTUNG IN EINEM MRT-SYSTEM**

DETERMINING THE POSITION OF A DEVICE IN AN MRI SYSTEM

DÉTERMINATION DE POSITION D'UN DISPOSITIF DANS UN SYSTÈME D'IRM

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(43) Veröffentlichungstag der Anmeldung:
**09.08.2023 Patentblatt 2023/32**

(73) Patentinhaber: **Siemens Healthineers AG**
**91301 Forchheim (DE)**

(72) Erfinder: **Popescu, Stefan**
**91056 Erlangen (DE)**

(74) Vertreter: **Siemens Healthineers**
**Patent Attorneys**
**Postfach 22 16 34**
**80506 München (DE)**

(56) Entgegenhaltungen:
WO-A1-00/13586    US-A1- 2003 184 297
US-A1- 2007 145 978    US-A1- 2021 212 588

- **JAMES A SMITH ET AL: "A Novel Position and Orientation Sensor for MRI", PROCEEDINGS OF THE INTERNATIONAL SOCIETY FOR MAGNETIC RESONANCE IN MEDICINE, ISMRM, 25TH ANNUAL MEETING AND EXHIBITION, HONOLULU, HAWAII, USA, 22-27 APRIL 2017, no. 3930, 7 April 2017 (2017-04-07), pages 3930, XP040691498**

**Beschreibung**

Die vorliegende Erfindung betrifft ein Verfahren zur Lagebestimmung einer Vorrichtung im Inneren eines Bildgebungsvolumens eines MRT-Systems, wobei das Bildgebungsvolumen von einem Feldmagneten zum Erzeugen eines statischen Grundmagnetfelds entlang einer Längsachse und von einer Gradientenspule des MRT-Systems umgeben ist. Die Erfindung ist ferner auf ein entsprechendes MRT-System gerichtet.

Magnetresonanztomographiesysteme, kurz MRT-Systeme, sind bildgebende Vorrichtungen, die ein starkes äußeres Magnetfeld nutzen, um die Kernspins eines zu untersuchenden Objekts auszurichten und sie durch Anlegen eines HF-Anregungspulses zur Präzession um die entsprechende Ausrichtung anzuregen. Die Präzession beziehungsweise der Übergang der Spins von diesem angeregten Zustand in einen Zustand mit geringerer Energie erzeugt als Antwort ein elektromagnetisches Wechselfeld, das über Empfangsantennen als MR-Signal detektiert werden kann.

Mit Hilfe von magnetischen Gradientenfeldern kann den Signalen eine Positionskodierung aufgeprägt werden, die es anschließend erlaubt, das erhaltene Signal einem Volumenelement des Untersuchungsobjekts zuzuordnen. Das empfangene Signal kann dann ausgewertet werden, um beispielsweise eine Bilddarstellung des Untersuchungsobjekts bereitzustellen.

Bei vielen MRT-Anwendungen ist es vorteilhaft, die Lage, insbesondere die Position und/oder Orientierung, einer Vorrichtung innerhalb des durch die Gradientenspule definierten Bildgebungsvolumens zu kennen, beispielweise in Bezug auf die Anatomie des Patienten. Die Lage des Patienten in dem Bildgebungsvolumen kann beispielsweise durch visuelle Marker oder dergleichen definiert oder bestimmt werden, sodass es wünschenswert ist, die Lage der Vorrichtung möglichst genau zu bestimmen. Bei der Vorrichtung kann es sich beispielsweise um lokalen MR-Empfangsspulen handeln, die direkt am Patienten angeordnet werden, etwa Kopfspulen, Kniespulen und so weiter. Es kann sich aber auch um ein Gerät zur medizinischen Behandlung des Patienten handeln, etwa einen Katheter, ein chirurgisches Instrument, eine Biopsienadel, einen Roboterarm, und so weiter, handeln.

Die Bewegung des Patienten während einer MRT-Untersuchung ist ein bekanntes Problem, insbesondere bei MRT-Untersuchungen, die längere Zeit in Anspruch nehmen. Die Patientenbewegung kann die gemessenen Signale verändern und Bildartefakte verursachen, die die Erkennung wesentlicher Merkmale, also insbesondere des radiologischen Befunds, verdecken oder behindern können. Besonders vor diesem Hintergrund ist die Bestimmung der Lage der Vorrichtung vorteilhaft.

Eine Methode zur Erkennung der Position einer Empfangsspule verwendet einen in die Spulenelektronik integrierten Hall-Sensor, der die lokale Intensität des statischen Magnetfelds misst. Außerhalb des Bildgebungsvolumens ist das statische Magnetfeld sehr inhomogen und weist starke statische Feldgradienten auf. Die Messwerte des Hall-Sensors können genutzt werden, um die Position der Empfangsspule zu ermitteln, wenn Empfangsspule und Patient vom Patententisch in das Bildgebungsvolumen hinein bewegt werden. Sobald sich die Empfangsspule jedoch innerhalb des durch ein sehr homogenes Magnetfeld gekennzeichneten Bildgebungsvolumens befindet, bleibt das Signal des Hall-Sensors im Wesentlichen konstant, selbst wenn sich die Spulenposition innerhalb des Bildgebungsvolumens verändert. Dies hat zur Folge, dass die Veränderung der Lage der Empfangsspule aufgrund von Patientenbewegungen unerkannt bleiben kann.

Bei anderen Verfahren wird eine Kamera verwendet, um die Position der Empfangsspule am Körper des Patienten zu erkennen, bevor der Patient mit dem Patententisch in das Bildgebungsvolumen gebracht wird. Eine weitere Kamera im Bildgebungsvolumen kann die Bewegung der Empfangsspule während der Untersuchung erkennen. Dies erfordert jedoch zwingend die freie Sicht zwischen der Kamera und der Empfangsspule, die aber beispielsweise durch Decken, sonstiges Zubehör, Stützelemente oder dielektrische Pads zur Verbesserung der HF-Umgebung oder die Gliedmaßen des Patienten, behindert werden kann.

Aus dem Dokument WO 00/13586 A1 ist ein Verfahren und eine Vorrichtung bekannt, um den Ort und eine Orientierung eines Objekts, beispielsweise eines medizinischen Instruments, im Inneren oder außerhalb eines Körpers zu bestimmen, während der Körper von einem Magnetresonanztomographen abgetastet wird. Insbesondere ermöglichen es das Verfahren und die Vorrichtung, die Position unterschiedlicher Vorrichtungen, z.B. Kathetern, chirurgischen Instrumenten, Biopsienadeln etc. durch Erfassen von Spannungen, die durch zeitvariable Magnetfelder in einem Satz Miniaturspulen induziert werden, zu ermitteln. Derartige zeitvariable Felder werden von dem Magnetresonanztomographen im normalen Betrieb erzeugt.

Das Dokument US 2021/212588 A1 beschreibt eine drahtlose Signalerfassungsvorrichtung, die drei zueinander orthogonale Erfassungsspulen aufweist, in die durch ein zeitvariables und ortsabhängiges Magnetfeld in einer Messkammer eines Magnetresonanzscanners eine Spannung induziert wird. Ein Hochfrequenzerfassungsschaltkreis erfasst Radiofrequenzpulse, die von einem Magnetresonanzscanner ausgesendet werden und ein drahtloser Übertragungsschaltkreis sendet die Daten von der Signalerfassungsvorrichtung aus. Ein 3-achsiges Magnetometer wird genutzt, um einen magnetischen Fluss in der Messkammer des Magnetresonanzscanners zu messen. Ein Prozessor nutzt die erfassten Radiofrequenzpulse, um die von dem Magnetometer und den Erfassungsspulen erfassten Messwerte mit einem Zeitrahmen der Gradienten-Ansteuerungshardware zu synchronisieren, wobei die Messwerte in einen PulssequenzVerlauf eingepasst werden. Der Prozessor kombiniert weiterhin Messungen von in den Erfassungsspulen induzierten Spannungen und dem magnetischen Fluss mit dem Pulssequenzverlauf, um die momentane Position und Ausrichtung der Signalerfassungsvorrichtung zu ermitteln.

Aus dem Dokument JAMES A SMITH ET AL: "A Novel Position and Orientation Sensor for MRI",PROCEEDINGS OF THE INTERNATIONAL SOCIETY FOR MAGNETIC RESONANCE IN MEDICINE, ISMRM, 25TH ANNUAL MEETING AND EXHIBITION, HONOLULU, HAWAII,USA, 22-27 APRIL 2017, Nr. 3930, 7. April 2017 (2017-04-07), Seite 3930, XP040691498 ist eine Vorrichtung bekannt, die eine Kopfhaltung in und in Bezug zu einem Scanner erfassen kann. Durch eine Kombination kommerziell verfügbarer 3-achsiger MEMS-Beschleunigungsmesser und einer anisotropen magnetoresistiven Messbrücke wird eine Ausrichtung der Vorrichtung mit einer mittleren quadratischen Abweichung von +/- 0,12 Grad überwacht, während eine Translationsbewegung mittels in eine einzelne Spule durch zeitvariable Magnetfeldgradienten induzierte Spannung mit einer Genauigkeit von +/- 0,45 mm abgeschätzt wird.

Das Dokument US 2003/184297 A1 beschreibt ein System zur Kombination einer elektromagnetischen Positions- und Ausrichtungsverfolgung mit einem Magnetresonanzscanner. Eine Ausführungsform weist einen Magnetresonanzscanner auf, der ein Referenzkoordinatensystem zur Abtastung eines Ziels vorgibt. Mit dem Magnetresonanzscanner ist eine Magnetfeldquelle gekoppelt, die ein Magnetfeld erzeugt. Das Magnetfeld wird von einem Magnetfeldsensor erfasst, der ein Signal proportional zu dem Magnetfeld erzeugt. Der Magnetfeldsensor hat eine Position relativ zu dem Referenzkoordinatensystem. Die Position des Magnetfeldsensors relativ zu dem Referenzkoordinatensystem des Magnetresonanzscanners wird von einer Positionsverfolgungseinheit unter Verwendung von zumindest einem Feldlinien-Segmentmodell der Magnetfeldquelle und dem Signal des Magnetfeldsensors ermittelt.

Aus dem Dokument US 2007/145978 A1 ist ein Magnetresonanzsystem zur Bilderfassung auf verschiedene Arten bekannt, wobei ein Patiententisch bewegt wird. Das Magnetresonanzsystem weist einen Patiententisch auf, der eine in eine vorbestimmte Richtung durch ein statisches Magnetfeld bewegliche Tischoberfläche hat, als auch eine Mehrzahl an Hochfrequenzempfangsspulen, die beispielsweise aus einer Mehrzahl an Spulengruppen bestehen. Die Tischoberfläche wird automatisch in Längsrichtung in Übereinstimmung mit einer Länge jeder Spulengruppe in die vorbestimmte Richtung bewegt. An jeder erreichten Position wird eine Bilderfassung mit einer gegebenen Pulssequenz durchgeführt. Ein Echosignal wird mit der Mehrzahl der Hochfrequenzspulen erfasst und dann von einer Eingangs-Schalteinheit an einen Empfangssystem-Schaltkreis weitergeleitet. Das Echosignal wird in dem Schaltkreis einer vorbestimmten Signalverarbeitung unterworfen, um in Echo-Daten umgewandelt zu werden. Aus den Echo-Daten wird von einem Host-Rechner eine Magnetresonanzabbildung erzeugt.

Es ist eine Aufgabe der vorliegenden Erfindung, die Lage einer Vorrichtung innerhalb des Bildgebungsvolumens eines MRT-Systems zuverlässig zu bestimmen.

Diese Aufgabe wird gelöst durch den jeweiligen Gegenstand der unabhängigen Ansprüche. Vorteilhafte Weiterbildungen und bevorzugte Ausführungsformen sind Gegenstand der abhängigen Ansprüche.

Die Erfindung beruht auf der Erkenntnis, dass eine Gradientenspule im Inneren der Gradientenspule und damit im Inneren des Bildgebungsvolumens ein Magnetfeld erzeugt, das neben Komponenten parallel zum Grundmagnetfeld stets auch signifikante Komponenten senkrecht dazu aufweist. Diese Komponenten werden erfindungsgemäß durch wenigstens eine Leiterschleife detektiert und zur Lagebestimmung der Leiterschleife und damit der Vorrichtung verwendet.

Gemäß einem Aspekt der Erfindung wird ein Verfahren zur Lagebestimmung einer Vorrichtung im Inneren eines Bildgebungsvolumens eines MRT-Systems gemäß Anspruch 1 angegeben. Das MRT-System weist dabei einen Feldmagneten zum Erzeugen eines statischen Grundmagnetfelds entlang einer Längsachse des MRT-Systems auf sowie eine Gradientenspule, welche das Bildgebungsvolumen umgeben. Die Vorrichtung weist wenigstens eine erste Leiterschleife auf, die innerhalb einer ersten Schleifenebene verläuft. Mittels der Gradientenspule wird ein magnetisches Wechselfeld in dem Bildgebungsvolumen erzeugt und mittels der wenigstens einen ersten Leiterschleife wird wenigstens ein erster Messwert bestimmt, der von einer ersten Induktionsspannung abhängt, die durch eine erste Komponente des Wechselfelds senkrecht zu der Längsachse in der wenigstens einen ersten Leiterschleife induziert wird. Eine Lage der Vorrichtung im Inneren des Bildgebungsvolumens wird abhängig von dem wenigstens einen ersten Messwert und einem vorgegebenen Magnetfeldmodell für die Gradientenspule wenigstens teilweise bestimmt.

Bei dem Bildgebungsvolumen handelt es sich insbesondere um einen Bereich innerhalb des MR-Scanners des MRT-Systems, insbesondere innerhalb des sogenannten Patiententunnels, der der auch als Bore bezeichnet wird, welcher im Wesentlichen durch die Gradientenspule und gegebenenfalls eine radial innerhalb der Gradientenspule angeordnete HF-Sendespule zum Senden hochfrequenter Wechselfelder definiert wird. Das Bildgebungsvolumen ist also insbesondere als Volumenbereich zu verstehen, innerhalb dessen ein Objekt prinzipiell abgebildet werden kann, beispielsweise wenn die HF-Sendespule auch als Empfangsspule genutzt wird. Wird eine lokale MR-Empfangsspule verwendet, so befindet sich diese insbesondere innerhalb des Bildgebungsvolumens. Das bedeutet, dass die lokale MR-Empfangsspule nicht das Bildgebungsvolumen definiert, sondern gegebenenfalls ein weiteres Bildgebungsvolumen im Inneren der lokalen MR-Empfangsspule.

Die wenigstens eine Leiterschleife ist Teil der lokalen MR-Empfangsspule, mit anderen Worten entspricht die Vorrichtung der lokalen MR-Empfangsspule. Durch die Bestimmung der Lage der Vorrichtung kann in diesem Fall auf die Lage weiterer Objekte, beispielsweise des Patienten oder eines Körperteils des Patienten oder eines medizinischen Werkzeugs, geschlossen werden, wenn dessen relative Lage zu der Vorrichtung entsprechend vorgegeben oder festgelegt ist.

Um die Lage der Vorrichtung wenigstens teilweise zu bestimmen, wird abhängig von dem wenigstens einen Messwert und dem Magnetfeldmodell insbesondere eine Lage der wenigstens einen Leiterschleife wenigstens teilweise bestimmt. Die Lage der wenigstens einen Leiterschleife kann gleich der Lage der Vorrichtung entsprechen oder die Lage der Vorrichtung kann aus der Lage der wenigstens einen Leiterschleife abgeleitet werden.

Die wenigstens eine Leiterschleife kann auch eine endliche Ausdehnung in einer Richtung senkrecht zur ersten Schleifenebene aufweisen. Dass die wenigstens eine Leiterschleife innerhalb der ersten Schleifenebene verläuft, kann derart aufgefasst werden, dass alle Leiterschleifen der wenigstens einen ersten Leiterschleife jeweils parallel zueinander und parallel zu der Schleifenebene sind.

Die Lage der wenigstens einen Leiterschleife und dementsprechend die Lage der Vorrichtung kann in einem vorgegebenen Koordinatensystem bestimmt werden, beispielsweise einem ortsfesten Koordinatensystem bezüglich der Gradientenspule. Die Längsachse des MRT-Systems kann beispielsweise als z-Richtung dieses Koordinatensystems aufgefasst werden und entsprechende zur z-Richtung senkrechte Richtungen als x- und y-Achse des Koordinatensystems. Es können aber auch andere Bezugssysteme gewählt werden, falls diese zur weiteren Verwendung vorteilhaft sind. Unter dem Begriff der Lage kann insbesondere die Kombination der dreidimensionalen Position und dreidimensionalen Orientierung im entsprechenden Bezugssystem verstanden werden. Mit anderen Worten kann die Lage auch als Pose bezeichnet werden. Beispielsweise kann die Lage durch dreidimensionale Koordinaten eines Referenzpunkts der Vorrichtung und drei Orientierungswinkel einer bestimmten Referenzrichtung der Vorrichtung gegeben sein. Um die Lage vollständig zu bestimmen, müssten also insbesondere die dreidimensionale Position und die dreidimensionale Orientierung der Vorrichtung bestimmt werden.

Dass die Lage der Vorrichtung in dem erfindungsgemäßen Verfahren wenigstens teilweise bestimmt wird, kann derart verstanden werden, dass die Lage entweder vollständig oder unvollständig bestimmt wird. Unter einer unvollständigen Bestimmung der Lage kann verstanden werden, dass beispielsweise nur einzelne Koordinaten der dreidimensionalen Position oder einzelne Winkel der dreidimensionalen Orientierung bestimmt werden, aber nicht alle drei Koordinaten der Position und alle drei Winkel der Orientierung. Alternativ kann die teilweise Bestimmung der Lage der Vorrichtung darin bestehen, dass eine oder mehrere Beschränkungen der sechs Freiheitsgrade, also der drei Koordinaten und der drei Orientierungsmittel, explizit oder implizit bestimmt werden, wobei die Beschränkung über die Anordnung der wenigstens einen ersten Leiterschleife innerhalb des Bildgebungsvolumens hinausgeht.

Beispielsweise kann also durch die wenigstens teilweise Bestimmung der Lage bestimmt werden, in welchem Abstand zum Zentrum des Bildgebungsvolumens, also zur Längsachse, sich der Referenzpunkt der Vorrichtung befindet, in welchem Winkelbereich sich die entsprechenden Orientierungsmittel befinden und so weiter. Ob die Lage vollständig oder nur teilweise bestimmt wird, beziehungsweise wie viel Information bezüglich der Lage der Vorrichtung bestimmt wird, hängt davon ab, ob und welche zusätzlichen Informationen neben dem wenigstens einen Messwert und dem Magnetfeldmodell zur Lagebestimmung zur Verfügung stehen. Es sei jedoch darauf hingewiesen, dass auch ohne solche zusätzlichen Informationen ausschließlich basierend auf dem wenigstens einen Messwert und dem Magnetfeldmodell eine teilweise Bestimmung der Lage möglich ist. Eine vollständige Bestimmung der Lage erfordert in der Regel jedoch weitere Informationen, die beispielsweise aufgrund baulicher Beschränkungen gegeben sein können oder durch weitere Sensorsysteme, wie etwa Hall-Sensoren, weitere Leiterschleifen, Kameras und so weiter bestimmt werden können.

Das Magnetfeldmodell kann beispielsweise das durch die Gradientenspule im Inneren des Bildgebungsvolumens erzeugte Magnetfeld in ortsaufgelöster Form und in allen drei Raumrichtungen oder jedenfalls in drei räumlichen Dimensionen, beinhalten, beispielsweise auch in ortsaufgelöster und zeitaufgelöster Form. Das Magnetfeldmodell hängt also insbesondere von den geometrischen und elektrischen Eigenschaften der Gradientenspule ab sowie gegebenenfalls von der Ansteuerung der Gradientenspule. Das Magnetfeldmodell kann beispielsweise durch Vermessung des Magnetfelds im Inneren des Bildgebungsvolumens und/oder durch Simulationen und/oder sonstige Berechnungen bestimmt werden und auf dem MRT-System, insbesondere wenigstens einer Auswerteeinheit des MRT-Systems, gespeichert werden.

Die Gradientenspule eines MRT-Systems ist so ausgelegt, dass im Inneren des Bildgebungsvolumens in erster Linie ein Magnetfeldgradient eines Magnetfelds in Richtung der Längsachse erzeugt werden kann. Der Magnetfeldgradient entspricht einer Änderung der Magnetfeldstärke in einer der drei Raumrichtungen. Ein MRT-System hat dabei in der Regel drei solcher Gradientenspulen, deren Magnetfeldstärke sich jeweils entlang einer anderen Raumrichtung verändert. Ein Magnetfeld, das ausschließlich entlang der Längsachse orientiert ist, ist jedoch nur theoretisch möglich, beispielsweise für unendlich große Spulen. In jeder tatsächlichen Implementierung einer Gradientenspule weist das durch diese erzeugte Magnetfeld im Inneren des Bildgebungsvolumens stets Komponenten in alle drei Raumrichtungen auf. Eine Ausnahme können möglicherweise ausgezeichnete Symmetriepunkte im exakten Zentrum des Bildgebungsvolumens sein, die jedoch ebenfalls nur von theoretischer Bedeutung sind. Diese Erkenntnis und die Ausnutzung dieser Erkenntnis zur Lagebestimmung der Vorrichtung im Bildgebungsvolumen eines MRT-Systems liegen der vorliegenden Erfindung zugrunde.

Insbesondere ist es aufgrund der skizzierten Umstände unabhängig von der Orientierung der Schleifenebene bezüglich der Längsachse immer möglich, eine entsprechenden Induktionsspannung oder einen entsprechenden Induktionsstrom zu messen und bei gleichzeitiger Kenntnis des Magnetfeldmodells Rückschlüsse auf die Lage der wenigstens einen Leiterschleife zu ziehen. Auf diese Weise kann die Komplexität der Mittel zum Bestimmen der Lage der Vorrichtung, also insbesondere die Komplexität der Vorrichtung selbst, reduziert werden.

Insbesondere ermöglicht es die Erfindung, dass eine lokale MR-Empfangsspule selbst zur Lagebestimmung der MR-Empfangsspule verwendet wird, obwohl die Leiterschleifen einer lokalen MR-Empfangsspule in der Regel parallel oder jedenfalls nicht senkrecht zu dem Grundmagnetfeld. Dies ermöglicht eine besonders vorteilhafte synergetische Kombination der grundlegenden Funktionalität der lokalen MR-Empfangsspule, nämlich der Detektion von MR-Signalen aus dem zu untersuchenden Objekt, mit der zusätzlichen Funktionalität der Lagebestimmung. Dabei sei darauf hingewiesen, dass die Empfangsspule nicht nur hochfrequente elektromagnetische Wechselfelder der Sendespule beziehungsweise der Kernspinresonanzsignale in Reaktion auf diese erfasst werden können, sondern auch die gegebenenfalls eine deutlich geringere Frequenz aufweisende Signale der Gradientenspule, die während der MRT-Untersuchung ohnehin zum Erzeugen der Magnetfeldgradienten erzeugt werden müssen. Es ist also nicht zwingend erforderlich, dass das magnetische Wechselfeld der Gradientenspule dediziert zur Lagebestimmung erzeugt wird. Dies ist zwar möglich, mit Vorteil können jedoch die ohnehin erzeugten Gradientenimpulse genutzt werden.

Ist die Lage der Vorrichtung bekannt oder zumindest eingegrenzt, so kann beispielsweise ein Benutzer des MRT-Systems automatisch darüber informiert werden, ob die Lage von einer gewünschten oder optimalen Lage abweicht. Darüber hinaus kann ein Algorithmus eingesetzt werden, der automatisch einen oder mehrere Scanparameter, wie beispielsweise einen Beschleunigungsfaktor R, eine Phasencodierungsrichtung und so weiter abhängig von der wenigstens teilweise bestimmten Lage der Vorrichtung so auswählt, dass die gegebene Lage oder, beispielsweise der Vorrichtung bezüglich des Patienten, optimal genutzt werden kann. Alternativ oder zusätzlich können auch Bildrekonstruktionsalgorithmen die wenigstens teilweise bestimmte Lage benutzen, um beispielsweise durch exaktere Abschätzung der Spulenempfindlichkeit der lokalen MR-Empfangsspule die resultierende Bildqualität zu verbessern.

Gemäß dem erfindungsgemäßen Verfahren wird abhängig von einem MR-Signal aus einem zu untersuchenden Objekt in dem Bildgebungsvolumen ein MR-Bild erzeugt.

Bei der Erzeugung des MR-Bildes kann die wenigstens teilweise bestimmte Lage der Vorrichtung automatisch oder manuell berücksichtigt werden. Beispielsweise kann eine MR-Aufnahme wiederholt oder teilweise wiederholt werden, wenn die Lage nicht einem vorgegebenen Erfordernis oder einer vorgegebenen Erwartung entspricht. Außerdem können automatisch Bewegungskompensationsalgorithmen abhängig von der wenigstens teilweise bestimmten Lage durchgeführt werden, um das MR-Bild zu erzeugen.

Zum Erzeugen des MR-Signals kann eine bekannte MR-Sequenz appliziert werden, wobei beispielsweise eine globale HF-Sendespule, welche das Bildgebungsvolumen umgibt, entsprechende HF-Impulse in das Bildgebungsvolumen einstrahlt und mittels der Gradientenspule eine Sequenz von Magnetfeldgradienten in dem Bildgebungsvolumen erzeugt wird. Dadurch wird eine Kernspinresonanz in dem zu untersuchenden Objekt hervorgerufen, und sich ergebende hochfrequente MR-Signale können dann mittels der HF-Sendespule, falls diese auch als Empfangsspule genutzt wird, und/oder mittels einer oder mehrerer weiteren lokalen MR-Empfangsspulen detektiert werden.

Gemäß dem erfindungsgemäßen Verfahren wird das MR-Signal aus dem zu untersuchenden Objekt in dem Bildgebungsvolumen mittels der wenigstens einen ersten Leiterschleife detektiert und das MR-Bild wird abhängig von dem MR-Signal erzeugt.

Mit anderen Worten wird die wenigstens eine erste Leiterschleife nicht nur zur Erzeugung des wenigstens einen Messwerts und damit zur wenigstens teilweisen Lagebestimmung der Vorrichtung verwendet, sondern auch als reguläre lokale MR-Empfangsspule. Dazu ist die Vorrichtung oder die wenigstens eine Leiterschleife insbesondere Teil einer lokalen MR-Empfangsspule.

Prinzipiell wird auch das MR-Signal über eine entsprechende Induktionsspannung in der wenigstens einen Leiterschleife detektiert. Die Induktionsspannungen aufgrund des MR-Signals und aufgrund des magnetischen Wechselfelds der Gradientenspule, die zur Bestimmung des wenigstens einen Messwerts herangezogen wird, können zeitlich oder in anderer Weise voneinander getrennt werden. Beispielsweise kann ausgenutzt werden, dass die Frequenz der von der Sendespule erzeugten Signale und dementsprechend die Frequenz der MR-Signale um ein Vielfaches höher ist als eine Frequenz der mittels der Gradientenspule erzeugten magnetischen Wechselfelder. Es kann also insbesondere eine Frequenzfilterung durchgeführt werden, um die Detektion des MR-Signals von der Detektion des durch die Gradientenspule erzeugten magnetischen Wechselfelds voneinander zu trennen. Die Frequenz des MR-Signals entspricht dabei insbesondere der entsprechenden Lamorfrequenz der zur Bildgebung verwendeten Atomkerne. Diese liegt beispielsweise in einem Frequenzbereich von 1 MHz bis 500 MHz, abhängig von der Grundmagnetfeldstärke des Feldmagneten des MRT-Systems. Die gepulsten Gradientenfelder der Gradientenspule, also das mittels der Gradientenspule erzeugte magnetische Wechselfeld, hat dagegen eine Frequenz im Bereich einiger kHz oder einiger 10 kHz.

Gemäß zumindest einer Ausführungsform, bei der das MR-Signal erfindungsgemäß mittels der wenigstens einen ersten Leiterschleife detektiert wird, wird zum Bestimmen des wenigstens einen ersten Messwerts das MR-Signal beziehungsweise die aus dem MR-Signal resultierende weitere Induktionsspannung oder ein entsprechendes Signal, unterdrückt, insbesondere mittels eines Filterschaltkreises.

Dadurch wird also erreicht, dass der wenigstens eine Messwert lediglich das magnetische Wechselfeld der Gradientenspule, nicht aber das MR-Signal widerspiegelt.

Andererseits kann zum Bestimmen oder Detektieren des MR-Signals die Induktionsspannung aufgrund des magnetischen Wechselfelds der Gradientenspule unterdrückt werden, beispielsweise mittels des Filterschaltkreises oder eines weiteren Filterschaltkreises.

So kann also sichergestellt werden, dass die Detektion des MR-Signals nicht durch das magnetische Wechselfeld der Gradientenspule beeinflusst wird.

Beispielsweise können der wenigstens eine erste Messwert und das MR-Signal durch unterschiedliche Messkanäle oder Empfangskanäle detektiert werden, wobei der Filterschaltkreis und/oder der weitere Filterschaltkreis in den entsprechenden Messkanälen implementiert ist.

Gemäß zumindest einer Ausführungsform wird die Vorrichtung derart in dem Bildgebungsvolumen positioniert, dass die Schleifenebene parallel zu der Längsachse ist, insbesondere wenigstens näherungsweise parallel zu der Längsachse ist.

Dabei kann die Schleifenebene beispielsweise als wenigstens näherungsweise parallel zur Längsachse betrachtet werden, wenn ein Winkel zwischen einer normalen Richtung, die senkrecht auf der Schleifenebene steht, und der Längsachse wenigstens näherungsweise gleich 90° ist, also beispielsweise größer als 60 Grad und kleiner als 120 Grad oder beispielsweise größer als 70° und kleiner als 110° oder beispielsweise größer als 80° und kleiner als 100°.

Dies kann insbesondere der Fall sein, wenn die Vorrichtung einer lokalen MR-Empfangsspule entspricht oder Teil einer solchen ist.

Gemäß zumindest einer Ausführungsform weist die Vorrichtung wenigstens eine zweite Leiterschleife auf, die innerhalb einer zweiten Schleifenebene verläuft, die verschieden von der ersten Schleifenebene ist. Mittels der wenigstens einen zweiten Leiterschleife wird wenigstens ein zweiter Messwert bestimmt, der von einer zweiten Induktionsspannung abhängt, die durch eine zweite Komponente des Wechselfelds senkrecht zu der Längsachse in der wenigstens einen zweiten Leiterschleife induziert wird. Die Lage der Vorrichtung wird abhängig von dem wenigstens einen Messwert, dem wenigstens einen zweiten Messwert und dem Magnetfeldmodell für die Gradientenspule wenigstens teilweise bestimmt.

Beispielsweise können in solchen Ausführungsformen die wenigstens eine erste und die wenigstens eine zweite Leiterschleife Teil einer lokalen MR-Empfangsspule sein.

Insbesondere wird die Vorrichtung derart in dem Bildgebungsvolumen positioniert, dass die zweite Schleifenebene wenigstens näherungsweise parallel zu der Längsachse ist und die erste Schleifenebene insbesondere ebenfalls wenigstens näherungsweise parallel zu der Längsachse ist.

Beispielsweise kann die relative Lage der wenigstens einen ersten Leiterschleife zu der wenigstens einen zweiten Leiterschleife bekannt oder fest vorgegeben sein. Zum wenigstens teilweisen Bestimmen der Lage der Vorrichtung kann also zum einen die Lage der wenigstens einen ersten Leiterschleife und die Lage der wenigstens einen zweiten Leiterschleife wie beschrieben bestimmt werden. Durch die vorgegebene oder bekannte relative Lage zueinander kann die Lage der Vorrichtung also dadurch genauer bestimmt werden beziehungsweise stärker eingeschränkt werden.

In entsprechenden Weiterbildungen kann eine Vielzahl von weiteren Leiterschleifen analog wie die wenigstens eine erste und die wenigstens eine zweite Leiterschleife verwendet werden, um eine möglichst genaue oder vollständige Bestimmung der Lage der Vorrichtung zu ermöglichen, wobei auch die weiteren Leiterschleifen beispielsweise Teil der lokalen MR-Empfangsspule sein können.

Gemäß zumindest einer Ausführungsform weist die Vorrichtung wenigstens eine dritte Leiterschleife auf, die innerhalb einer dritten Schleifenebene verläuft. Mittels der wenigstens einen dritten Leiterschleife wird wenigstens ein dritter Messwert bestimmt, der von einer dritten Induktionsspannung abhängt, die durch eine dritte Komponente des Wechselfelds senkrecht zu der Längsachse in der wenigstens einen dritten Leiterschleife induziert wird. Eine erste Lage der wenigstens einen ersten Leiterschleife im Inneren des Bildgebungsvolumens wird abhängig von dem wenigstens einen ersten Messwert und dem Magnetfeldmodell wenigstens teilweise bestimmt und eine dritte Lage der wenigstens einen dritten Leiterschleife im Inneren des Bildgebungsvolumens wird abhängig von dem wenigstens einen dritten Messwert und dem Magnetfeldmodell wenigstens teilweise bestimmt. Eine relative Lage der wenigstens einen dritten Leiterschleife bezüglich der wenigstens einen ersten Leiterschleife wird abhängig von der ersten Lage und abhängig von der dritten Lage bestimmt.

In solchen Ausführungsformen ist also insbesondere die relative Lage der dritten und der ersten Leiterschleifen vorab nicht oder nicht genau bekannt, kann aber in der beschriebenen Weise bestimmt oder näherungsweise bestimmt werden. Solche Ausführungsformen sind ebenfalls insbesondere dann vorteilhaft, wenn die lokale MR-Empfangsspule die wenigstens eine erste Leiterschleife und die wenigstens eine dritte Leiterschleife beinhaltet und beispielsweise als flexible Oberflächenspule ausgestaltet ist. Solche flexiblen Oberflächenspulen passen sich gegebenenfalls an die Oberfläche des Patienten oder dergleichen an, sodass die einzelnen Leiterschleifen eine per se nicht bekannte Orientierung zueinander aufweisen. In der genannten Weise kann die Lage beziehungsweise die Form der Oberfläche der flexiblen Oberflächenspule bestimmt werden.

Gemäß einem weiteren Aspekt der Erfindung wird auch ein MRT-System gemäß Anspruch 7 angegeben, das einen Feldmagneten zum Erzeugen eines statischen Grundmagnetfelds entlang einer Längsachse und eine Gradientenspule aufweist, wobei der Feldmagnet und die Gradientenspule ein Bildgebungsvolumen des MRT-Systems umgeben. Das MRT-System weist eine Vorrichtung mit wenigstens einer ersten Leiterschleife auf, wobei die wenigstens eine erste Leiterschleife innerhalb einer ersten Schleifenebene verläuft. Das MRT-System weist eine Steuereinheit auf, die dazu eingerichtet ist, die Gradientenspule anzusteuern, ein magnetisches Wechselfeld in dem Bildgebungsvolumen zu erzeugen. Das MRT-System weist eine Messeinheit auf, die mit der wenigstens einen ersten Leiterschleife verbunden ist. Die Messeinheit ist dazu eingerichtet, abhängig von einer ersten Induktionsspannung, die durch eine Komponente des

Wechselfelds senkrecht zu der Längsachse in der wenigstens einen ersten Leiterschleife induziert wird, wenigstens einen ersten Messwert zu bestimmen. Das MRT-System weist wenigstens eine Auswerteeinheit auf, die dazu eingerichtet ist, eine Lage der Vorrichtung im Inneren des Bildgebungsvolumens abhängig von dem wenigstens einen ersten Messwert und einem vorgegebenen Magnetfeldmodell für die Gradientenspule wenigstens teilweise zu bestimmen.

Die Steuereinheit, die Messeinheit und/oder die wenigstens eine Auswerteeinheit können in verschiedenen Ausgestaltungsformen separat zueinander vorgesehen werden oder auch teilweise oder vollständig kombiniert sein.

Die erfindungsgemäße Vorrichtung weist eine Abstimmkapazität auf, insbesondere einen Abstimmkondensator, die beziehungsweise der zwischen einem ersten Anschluss der wenigstens einen ersten Leiterschleife und einem zweiten Anschluss der wenigstens einen ersten Leiterschleife angeordnet ist. Die Vorrichtung weist ein induktives Bauelement auf, das elektrisch parallel zu der Abstimmkapazität angeordnet ist.

Eine solche Ausführungsform der Vorrichtung ist insbesondere vorteilhaft, wenn die Vorrichtung Teil der lokalen MR-Empfangsspulenanordnung ist oder gleich der lokalen MR-Empfangsspulenanordnung ist.

Gemäß zumindest einer Ausführungsform weist das MRT-System eine lokale MR-Empfangsspulenanordnung auf, welche die Vorrichtung enthält, wobei die lokale Empfangsspulenanordnung als flexibles Oberflächenspulenarray ausgestaltet ist.

Die wenigstens eine erste Leiterschleife entspricht dann einer Oberflächenspule des Oberflächenspulenarrays.

Gemäß zumindest einer Ausführungsform weist das MRT-System ein Gerät zur medizinischen Behandlung eines Patienten auf, wobei die wenigstens eine erste Leiterschleife und das Gerät eine vorgegebene räumliche Lage zueinander aufweisen.

Bei dem Gerät kann es sich beispielsweise um eine Biopsienadel, einen Katheter, ein chirurgisches Instrument, einen Roboterarm und so weiter handeln.

Die Induktionsspannung liegt insbesondere zwischen dem ersten und dem zweiten Anschluss der wenigstens einen Leiterschleife an.

Die Abstimmkapazität kann als Abstimmkondensator, also als entsprechendes elektronisches Bauelement, oder als parasitäre Kapazität zwischen Leitersegmenten der wenigstens einen ersten Leiterschleife realisiert sein.

Beispielsweise kann die Vorrichtung mehrere Abstimmkapazitäten aufweisen, die zwischen dem ersten und dem zweiten Anschluss angeordnet sind. In diesem Fall weist die Vorrichtung insbesondere für jede Abstimmkapazität ein entsprechendes zugeordnetes induktives Bauelement auf, das elektrisch parallel zu der Abstimmkapazität geschaltet ist.

Ohne das induktive Bauelement wäre die wenigstens eine Leiterschleife aufgrund der Abstimmkapazität für Gleichstrom insbesondere nichtleitend beziehungsweise hätte eine sehr hohe Impedanz bezüglich niederfrequenter Wechselströme. Durch das induktive Bauelement wird die Leitfähigkeit bei niedrigen Frequenzen erhöht. Hinsichtlich der hochfrequenten MR-Signale wirkt die Induktivität des induktiven Bauelements dagegen effektiv als Widerstand, sodass aufgrund der Parallelschaltung zu der Abstimmkapazität keine wesentliche Beeinflussung bei der Detektion des MR-Signals erfolgt.

In verschiedenen Ausführungsformen kann die Vorrichtung auch eine Verstimmkapazität, insbesondere einen Verstimmkondensator, aufweisen, der beziehungsweise die zwischen dem ersten und dem zweiten Anschluss angeordnet ist. Die Vorrichtung weist in diesem Fall ein weiteres induktives Bauelement auf, das elektrisch parallel zu der Verstimmkapazität geschaltet ist. So wirkt sich auch die Verstimmkapazität bei niedrigen Frequenzen nicht oder kaum aus, wohingegen sich das weitere induktive Bauelement bei hohen Frequenzen nicht oder nicht wesentlich auswirkt.

Gemäß zumindest einer Ausführungsform weist die Messeinheit einen Verstärker auf, der mit dem ersten Anschluss und dem zweiten Anschluss verbunden ist, insbesondere direkt oder indirekt, und dazu eingerichtet ist, an einem Ausgang des Verstärkers, der mit der wenigstens einen Auswerteeinheit verbunden ist, den wenigstens einen Messwert bereitzustellen.

Insbesondere weist der Verstärker einen ersten und einen zweiten Eingang auf, wobei der erste Eingang mit dem ersten Anschluss und der zweite Eingang mit dem zweiten Anschluss verbunden sind.

Gemäß zumindest einer Ausführungsform weist die Messeinheit oder die Vorrichtung einen weiteren Verstärker auf, der ebenfalls mit dem ersten und dem zweiten Anschluss verbunden ist und dazu eingerichtet ist, an einem Ausgang des weiteren Verstärkers, der ebenfalls mit der wenigstens einen Auswerteeinheit verbunden ist, das MR-Signal oder ein von dem MR-Signal abhängiges Messsignal bereitzustellen. Der Verstärker und der weitere Verstärker können also insbesondere entsprechende Teile eines ersten und eines zweiten Messkanals sein, die unterschiedliche mit der wenigstens einen Leiterschleife erfasste Ströme detektieren.

Gemäß zumindest einer Ausführungsform weist die Messeinheit einen Filterschaltkreis auf, der zwischen dem ersten Anschluss und einem ersten Eingang des Verstärkers sowie zwischen dem zweiten Anschluss und einem zweiten Eingang des Verstärkers angeordnet ist. Der Filterschaltkreis ist dazu ausgelegt, ein mittels der wenigstens einen Leiterschleife erfasstes MR-Signal zu unterdrücken.

Der Filterschaltkreis weist insbesondere einen ersten Eingang auf, der mit dem ersten Anschluss verbunden ist, und einen ersten Ausgang, der mit dem ersten Eingang des Verstärkers verbunden ist. Des Weiteren weist der Filterschaltkreis insbesondere einen zweiten Eingang auf, der mit dem zweiten Anschluss verbunden ist, und einen zweiten Ausgang, der mit dem zweiten Eingang des Verstärkers verbunden ist.

Der Filterschaltkreis kann beispielsweise als Tiefpassfilter oder Bandpassfilter ausgestaltet sein. Jedenfalls ist der Filterschaltkreis derart auf die Gradientenspule, das Grundmagnetfeld beziehungsweise die Ansteuerung der Gradientenspule abgestimmt, dass er Frequenzen, wie sie dem magnetischen Wechselfeld der Gradientenspule entsprechen, im Wesentlichen passieren lässt, wohingegen er Frequenzen, wie sie dem MR-Signal entsprechen, im Wesentlichen unterdrückt.

Beispielsweise kann die Vorrichtung einen weiteren Filterschaltkreis aufweisen, der zwischen dem ersten Anschluss und einem ersten Eingang des weiteren Verstärkers sowie zwischen dem zweiten Anschluss und einem zweiten Eingang des weiteren Verstärkers angeordnet ist. Der weitere Filterschaltkreis ist dazu ausgelegt, das durch das magnetische Wechselfeld der Gradientenspule erzeugte Signal in der wenigstens einen ersten Leiterschleife zu unterdrücken.

Der zweite Filterschaltkreis kann beispielsweise als Hochpassfilter oder als weiterer Bandpassfilter ausgestaltet sein. Der weitere Filterschaltkreis ist also insbesondere komplementär zum Filterschaltkreis ausgelegt. Insbesondere kann der erste Anschluss der wenigstens einen Leiterschleife mit einem ersten Eingang des weiteren Filterschaltkreises verbunden sein und der zweite Anschluss der wenigstens einen Leiterschleife mit einem zweiten Eingang des weiteren Filterschaltkreises. Ein erster Ausgang des weiteren Filterschaltkreises ist beispielsweise mit einem ersten Eingang des weiteren Verstärkers verbunden und ein zweiter Ausgang des weiteren Filterschaltkreises mit einem zweiten Eingang des weiteren Verstärkers.

Weitere Ausführungsformen des erfindungsgemäßen MRT-Systems folgen unmittelbar aus den verschiedenen Ausgestaltungen des erfindungsgemäßen Verfahrens und umgekehrt. Insbesondere lassen sich einzelne Merkmale und entsprechende Erläuterungen bezüglich der verschiedenen Ausführungsformen zu dem erfindungsgemäßen Verfahrens analog auf entsprechende Ausführungsformen des erfindungsgemäßen MRT-Systems übertragen und umgekehrt. Insbesondere ist das erfindungsgemäße MRT-System zum Durchführen eines erfindungsgemäßen Verfahrens ausgebildet oder programmiert. Insbesondere führt das erfindungsgemäße MRT-System das erfindungsgemäße Verfahren durch.

Gemäß einem weiteren Aspekt, der nicht in den durch die Ansprüche definierten Schutzumfang der Erfindung fällt, wird auch eine Vorrichtung zur Lagebestimmung für ein MRT-System angegeben. Die Vorrichtung weist wenigstens eine erste Leiterschleife auf, die innerhalb einer ersten Schleifenebene verläuft. Die Vorrichtung weist außerdem eine Messeinheit auf, die mit der wenigstens einen Leiterschleife verbunden ist und dazu eingerichtet ist, abhängig von einer ersten Induktionsspannung, die in der wenigstens einen ersten Leiterschleife induziert wird, wenigstens einen ersten Messwert zu bestimmen.

Gemäß zumindest einer Ausführungsform der Vorrichtung weist diese die Abstimmkapazität und das induktive Bauelement wie oben beschrieben auf.

Weitere Ausführungsformen der Vorrichtung folgen aus den verschiedenen Ausführungsformen des erfindungsgemäßen MRT-Systems sowie des erfindungsgemäßen Verfahrens.

Unter einer Recheneinheit kann insbesondere ein Datenverarbeitungsgerät verstanden werden, die einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "applicationspecific integrated circuit"), eines oder mehrere feldprogrammierbare Gate-Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere digitale Signalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer Festwertspeicher, EPROM (englisch: "erasable read-only memory"), als elektrisch löschbarer Festwertspeicher, EEPROM (englisch: "electrically erasable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Die wenigstens eine Auswerteeinheit, die Steuereinheit und/oder die Messeinheit des erfindungsgemäßen MRT-Systems kann eine oder mehrere Recheneinheiten gemäß diesem Verständnis beinhalten oder eine oder mehrere Recheneinheiten des MRT-Systems können die wenigstens eine Auswerteeinheit, die Steuereinheit und/oder die Messeinheit beinhalten.

Ist im Rahmen der vorliegenden Offenbarung die Rede davon, dass eine Komponente des erfindungsgemäßen MRT-Systems, insbesondere die Steuereinheit, die Messeinheit oder wenigstens eine Auswerteeinheit des MRT-Systems dazu eingerichtet, ausgebildet, ausgelegt, oder dergleichen ist, eine bestimmte Funktion auszuführen oder zu realisieren, eine bestimmte Wirkung zu erzielen oder einem bestimmten Zweck zu dienen, so kann dies derart verstanden werden, dass die Komponente, über die prinzipielle oder theoretische Verwendbarkeit oder Eignung der Komponente für diese Funktion, Wirkung oder diesen Zweck hinaus, durch eine entsprechende Anpassung, Programmierung, physische Ausgestaltung und so weiter konkret und tatsächlich dazu in der Lage ist, die Funktion auszuführen oder zu realisieren, die Wirkung zu erzielen oder dem Zweck zu dienen.

Eine Verbindung zweier elektrischer oder elektronischer Komponenten kann, sofern nicht ausdrücklich anders angegeben, derart verstanden werden, dass eine elektrische Verbindung zwischen den Komponenten besteht oder durch Betätigung eines oder mehrerer Schaltelemente hergestellt werden kann.

Insbesondere können die Komponenten direkt oder indirekt miteinander verbunden sein, sofern nicht anders angegeben. Dabei kann eine direkte Verbindung so verstanden werden, dass außer dem optionalen einen oder mehreren Schaltelementen keine weiteren elektrischen oder elektronischen Komponenten zwischen den Komponenten angeordnet sind, während eine indirekte Verbindung so verstanden werden kann, dass zusätzlich zu dem optionalen einen oder mehreren Schaltelementen eine oder mehrere weitere elektrische oder elektronische Komponenten, etwa Widerstände, Kondensatoren, Spulen und so weiter, zwischen den Komponenten angeordnet sind.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

Der Schutzumfang der Erfindung wird jedoch generell durch die Ansprüche bestimmt.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt. In den Figuren zeigen:

FIG 1      eine schematische Darstellung einer beispielhaften Ausführungsform eines erfindungsgemäßen MRT-Systems;

FIG 2      eine schematische Darstellung eines Magnetfelds;

FIG 3      eine schematische Darstellung einer Vorrichtung einer weiteren beispielhafte Ausführungsform eines erfindungsgemäßen MRT-Systems;

FIG 4      eine schematische Darstellung einer Vorrichtung einer weiteren beispielhafte Ausführungsform eines erfindungsgemäßen MRT-Systems;

FIG 5      eine schematische Darstellung einer Vorrichtung einer weiteren beispielhafte Ausführungsform eines erfindungsgemäßen MRT-Systems;

FIG 6      eine schematische Darstellung einer Vorrichtung einer weiteren beispielhafte Ausführungsform eines erfindungsgemäßen MRT-Systems;

FIG 7      eine schematische Darstellung einer Vorrichtung einer weiteren beispielhafte Ausführungsform eines erfindungsgemäßen MRT-Systems;

FIG 8      eine schematische Darstellung einer Vorrichtung einer weiteren beispielhafte Ausführungsform eines erfindungsgemäßen MRT-Systems;

FIG 9      eine schematische Darstellung einer Vorrichtung einer weiteren beispielhafte Ausführungsform eines erfindungsgemäßen MRT-Systems; und

FIG 10      eine schematische Darstellung möglicher Grundformen einer lokalen MR-Empfangsspule.

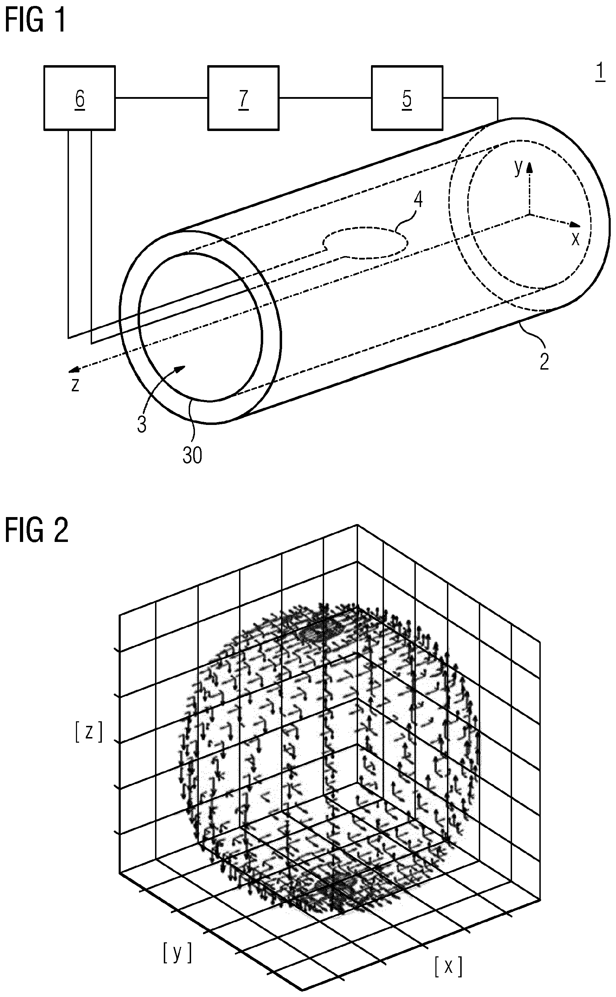

In FIG 1 ist schematisch eine beispielhafte Ausführungsform eines erfindungsgemäßen MRT-Systems 1 dargestellt.

Das MRT-System 1 weist eine Feldmagneten (nicht gezeigt) mit auf, der ein statisches Magnetfeld zur Ausrichtung von Kernspins einer Probe, beispielsweise eines Patienten, in einem Bildgebungsvolumen 3 in z-Richtung erzeugt, die als Längsachse des MRT-Systems bezeichnet werden kann. Das Bildgebungsvolumen 3 zeichnet sich durch ein sehr homogenes statisches Magnetfeld in z-Richtung auf. Bei dem Feldmagneten kann es sich beispielsweise um einen supraleitenden Magneten handelt, der magnetische Felder mit einer magnetischen Flussdichte von bis zu 3 T oder mehr bereitstellen kann. Für geringere Feldstärken können jedoch auch Permanentmagnete oder Elektromagnete mit normalleitenden Spulen Verwendung finden.

Weiterhin weist das MRT-System 1 eine Gradientenspule 2 auf sowie eine Steuereinheit 5 zur Ansteuerung der Gradientenspule 2, die dazu ausgelegt sind, zur räumlichen Differenzierung der erfassten Abbildungsbereiche in dem Bildgebungsvolumen 3 dem statischen Magnetfeld Magnetfelder zu überlagern, deren Betrag sich ortsabhängig entlang allen drei Raumrichtungen x, y, z verändern kann. Die Gradientenspule 2 kann beispielsweise als Spule aus normalleitenden Drähten ausgestaltet sein.

Das MRT-System 1 kann als Sendeantenne beispielsweise eine Körperspule 30 aufweisen, die dazu ausgelegt ist, ein über eine Signalleitung zugeführtes Hochfrequenzsignal in das Bildgebungsvolumen 3 abzustrahlen.

Die Steuereinheit 5 kann die Gradientenspule 2 und die Körperspule 30 mit verschiedenen Signalen versorgen. Die Steuereinheit 5 kann beispielsweise eine Gradientensteuerung aufweisen, die dazu ausgelegt ist, die Gradientenspule 2 über Zuleitungen mit variablen Strömen zu versorgen, welche zeitlich koordiniert die gewünschten Gradientenfelder in dem Bildgebungsvolumen 3 bereitstellen können.

Die Steuereinheit 5 kann auch eine Hochfrequenzeinheit aufweisen, die dazu ausgelegt ist, Hochfrequenzpulse oder Anregungspulse mit vorgegebenen zeitlichen Verläufen, Amplituden und spektraler Leistungsverteilung zur Anregung einer Magnetresonanz der Kernspins in dem Patienten zu erzeugen. Dabei können Pulsleistungen im Bereich von Kilowatt eingesetzt werden. Die Anregungspulse können über die Körperspule 30 oder über eine oder mehrere lokale Sendeantennen in den Patienten abgestrahlt werden. Die Steuereinheit 5 kann auch eine Steuerung enthalten, die über einen Signalbus mit der Gradientensteuerung und der Hochfrequenzeinheit kommunizieren kann.

Die Körperspule 30 kann in manchen Ausführungsformen auch dazu genutzt werden, von dem Patienten emittierte Resonanzsignale, auch als MR-Signale bezeichnet, zu empfangen und über eine Signalleitung abzugeben. Die Körperspule 30 kann in solchen Ausführungsformen also sowohl als Empfangsantenne sowie als Sendeantenne dienen.

Optional kann in der unmittelbaren Umgebung des Patienten eine lokale MR-Empfangsspule (nicht gezeigt), auch als Lokalspule bezeichnet, des MRT-Systems 1 angeordnet sein, die über eine Anschlussleitung mit einer Messeinheit 6 verbunden sein kann. Die Messeinheit 6 kann auch Teil der Steuereinheit 5 sein. Je nach Ausgestaltungsform kann die Lokalspule alternativ oder zusätzlich zur Körperspule 30 als Empfangsantenne dienen.

Das MRT-System 1 weist außerdem eine Auswerteeinheit 7 auf, die mit der Steuereinheit 5 verbunden ist, insbesondere mit der Hochfrequenzeinheit. Die Auswerteeinheit 7 kann die MR-Signale auswerten und basierend darauf nach bekannten Methoden ein MR-Bild rekonstruieren. Die Steuereinheit 5 kann auch Teil der Auswerteeinheit 7 sein.

Das MRT-System 1 weist eine Vorrichtung mit wenigstens einer Leiterschleife 4 auf, die innerhalb einer Schleifenebene verläuft und insbesondere derart in dem Bildgebungsvolumen 3 angeordnet ist, dass die Schleifenebene im Wesentlichen parallel zu der z-Richtung orientiert ist.

Die Gradientenspule 2 erzeugt, wie beschrieben, angesteuert durch die Steuereinheit 5 ein magnetisches Wechselfeld in dem Bildgebungsvolumen **3.** Dieses magnetische Wechselfeld weist im Allgemeinen Magnetfeldkomponenten in alle drei Raumrichtungen **x,** y, z auf. Demzufolge wird in der wenigstens einen Leiterschleife 4 eine Induktionsspannung hervorgerufen, auch wenn die Schleifenebene im Wesentlichen parallel zu der z-Richtung orientiert ist.

Die Messeinheit 6 ist mit der wenigstens einen Leiterschleife 4 verbunden und dazu eingerichtet, abhängig von der Induktionsspannung wenigstens einen Messwert zu bestimmen. Die Auswerteeinheit 7 ist dazu eingerichtet, eine Lage der Vorrichtung im Inneren eines Bildgebungsvolumens 3 abhängig von dem wenigstens einen Messwert und einem vorgegebenen Magnetfeldmodell für die Gradientenspule 2 wenigstens teilweise zu bestimmen.

In Ausführungsformen mit einer lokalen MR-Empfangsspule, kann diese die Vorrichtung oder die wenigstens einen Leiterschleife 4 beinhalten. Die Lage der Vorrichtung entspricht dann also der Lage der lokalen MR-Empfangsspule.

Ein Beispiel für das Magnetfeldmodell ist in FIG 2 schematisch dargestellt. Entgegen der üblichen vereinfachenden Annahme, sind die von der Gradientenspule 2 im Bildgebungsvolumen 3 erzeugten Gradientenfelder nicht exakt parallel zur Richtung des statischen Magnetfeldes, also der z-Richtung, ausgerichtet. Stattdessen erzeugt die Gradientenspule 2 zusätzliche Feldkomponenten, die orthogonal zu z also entlang der x- oder y-Achse ausgerichtet sind und deren Amplitude auch mit der z-Komponente vergleichbar ist. Da die Komponenten des Wechselfelds entlang der x- oder y-Achse sehr viel kleiner sind als das statische Grundmagnetfeld, können diese Komponenten für die reguläre MRT-Bildgebung vernachlässigt werden. Aus diesem Grund wurden die Komponenten des Wechselfelds entlang der x- und y-Achse in der Vergangenheit auch für andere Anwendungsmöglichkeiten nicht in Betracht gezogen. Da sie, im Gegensatz zum statischen Grundmagnetfeld, zeitabhängig sind, tragen sie jedoch signifikant zur Induktion in der Leiterschleife 4 bei und können daher gemäß der Erfindung zur Lagebestimmung genutzt werden.

FIG 2 zeigt beispielhaft die kartesischen Komponenten des Magnetfeldes, wie sie im Inneren der Gradientenspule 2 erfasst werden, die im statischen Modus betrieben wird, also mit konstantem Strom, wobei kein Grundmagnetfeld des Feldmagneten vorliegt. An jeder Abtaststelle wurden drei Feldwerte, die den drei orthogonalen Feldkomponenten Bx, By und Bz entsprechen, mit einem Vektormagnetometer gemessen, das an einem Roboterarm angebracht war und nacheinander an 480 räumlichen Positionen positioniert wurde, die über die Oberfläche einer Kugel verteilt sind. Auf der Grundlage dieser Messwerte kann mit Hilfe eines kalibrierten Magnetfeldmodells das Magnetfeld an einem beliebigen Ort innerhalb des Bildgebungsvolumens 3 berechnet werden. Die wenigstens eine Leiterschleife 4, die sich im Bildgebungsvolumen 3 des MRT-Systems 1 befindet, erfasst also ein durch gepulste Gradientenfelder induziertes Signal, auch wenn die Schleifenebene im Wesentlichen parallel zur z-Richtung orientiert ist.

Durch die Erfindung ist es zur Bestimmung der Lage einer lokalen MR-Empfangsspule nicht zwingend erforderlich, zusätzliche Sensoren einzusetzen. Stattdessen können die bereits vorhandenen Leiterschleifen der lokalen MR-Empfangsspule sowohl zur Detektion der schwachen hochfrequenten MRT-Signale als auch zur Detektion der durch die Gradientenpulse induzierten Signale im niederfrequenten Bereich eingesetzt werden.

Die Spannung, die in einer Leiterschleife induziert wird, wenn sich der magnetische Fluss durch den von der Leiterschleife umschlossenen Bereich ändert ergibt sich durch Integration der Änderung des magnetischen Vektorfeldes B über die von der Schleife umschlossene Fläche A, also durch Anwendung des Faraday'schen Induktionsgesetzes:

$$U= \int \frac{d\vec{B}}{dt} \cdot d\vec{s}$$

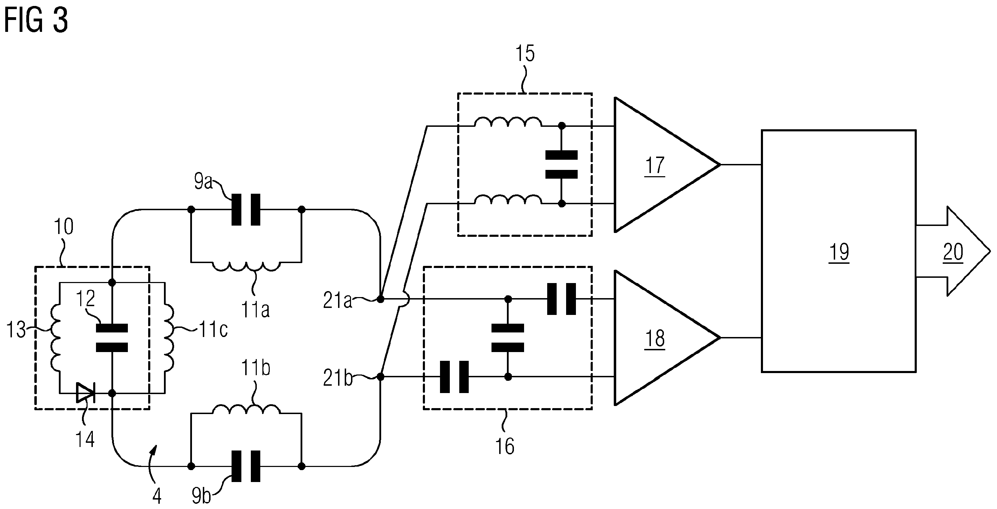

In FIG 3 bis FIG 5 sind schematische Implementierungen der Vorrichtung mit der wenigstens einen Leiterschleife 4 für verschiedene beispielhafte Ausführungsformen des erfindungsgemäßen MRT-Systems 1, beispielsweise des MRT-Systems 1 aus FIG 1, gezeigt.

Durch diese Vorrichtungen ist das MRT-System 1 dazu in der Lage, gleichzeitig die hochfrequenten MR-Signale und die durch pulsierende Gradientenfelder induzierten niederfrequenten Signale zur Lagebestimmung zu empfangen.

Eine Mehrkanal-MR-Empfangsspule kann beispielsweise als zweidimensionales flexibles Array ausgestaltet sein, das aus mehreren Empfangselementen besteht, etwa 2 bis 32 oder sogar 64 Empfangselemente. In FIG 3 bis FIG 5 ist ein solches Empfangselement dargestellt. Das Empfangselement weist die wenigstens eine Leiterschleife 4, beispielsweise als wenigstens eine Kupferschleife ausgeführt, auf sowie Abstimmmittel, beispielsweise Abstimmkondensatoren 9a, 9b, die zwischen einem ersten Anschluss 21a und einem zweiten Anschluss 21b der wenigstens einen Leiterschleife 4 angeordnet sind. Es können auch Verstimmungsmittel 10 vorgesehen sein, die beispielsweise einen Verstimmungskondensator 12 und eine parallel dazu angeordnete Reihenschaltung mit einer Verstimmungsinduktivität 13 und einer Diode 14 enthält.

Ferner kann eine Vorverstärkerschaltung 18 vorgesehen sein, die über eingangsseitig eine Anpassungsschaltung 16 mit den Anschlüssen 21a, 21b verbunden ist und ausgangsseitig mit einem Analog-Digital-Wandler 19, der über einen Datenbus 20 mit der Auswerteeinheit 7 oder einem Computer verbunden sein kann. Die Abstimmkondensatoren 9a, 9b sind beispielsweise entlang der wenigstens einen Leiterschleife 4 verteilt, um die elektrischen Felder zu reduzieren, die anderenfalls über lange Leitersegmente entstehen und gegebenenfalls zu dielektrischen Verlusten und damit einem reduzierten Signalzu-Rausch-Verhältnis führen können. Die Kapazitäten der Abstimmkondensatoren 9a, 9b sind insbesondere derart abgestimmt, sie mit der Induktivität der wenigstens einen Leiterschleife 4 bei der Larmor-Resonanzfrequenz des MRT-Systems 1 in Resonanz gehen, die je nach Feldstärke eine hohe Frequenz beispielsweise im Bereich von 1 MHz bis 500 MHz sein kann.

Parallel zu den Abstimmkondensatoren 9a, 9b und zu dem Verstimmungskondensator 12 ist jeweils ein induktives Bauelement 11a, 11b, 11c, sodass auch die durch die gepulsten Gradientenfelder induzierten niederfrequenten Signale im Bereich von einigen kHz erfasst werden können. Die Induktivität induktiven Bauelemente 11a, 11b, 11c wird derart gewählt, dass diese für die induzierten hochfrequenten MR-Signale eine hohe Impedanz aufweisen und praktisch einem offenen Stromkreis entsprechen. Im Gegensatz dazu ist die elektrische Impedanz der induktiven Bauelemente 11a, 11b, 11c bei niedrigen Frequenzen im Wesentlichen äquivalent zu einem Kurzschluss, der wenigstens eine Leiterschleife 4 für die durch die pulsierenden Gradientenfelder induzierten Signale schließt. Die Werte dieser Induktivitäten können abhängig von der Larmor-Frequenz beispielsweise im Bereich von einigen hundert $\mu H$ bis zu vielen mH liegen.

In manchen Ausführungsformen kann ein Signalvorverstärker 17 eingangsseitig über einen Filterschaltkreis 15, der beispielsweise als Tiefpassfilter ausgeführt sein kann, mit den Anschlüssen 21a, 21b und ausgangsseitig mit einem weiteren Eingang des Analog-Digital-Wandlers 19 oder mit einem weiteren Analog-Digital-Wandler (nicht gezeigt) verbunden sein. Die durch die gepulsten Gradienten induzierten und von der wenigstens einen Leiterschleife 4 erfassten Signale können dann über den Datenbus 20 aus gelesen und von Signalverarbeitungsalgorithmen weiter verwendet werden, um die Informationen über die Lage der wenigstens einen Leiterschleife 4 zu extrahieren. Analog kann auch die Lage weiterer Empfangselemente bestimmt werden und somit kann die Form der flexiblen Mehrkanal-MR-Empfangsspule beschrieben werden.

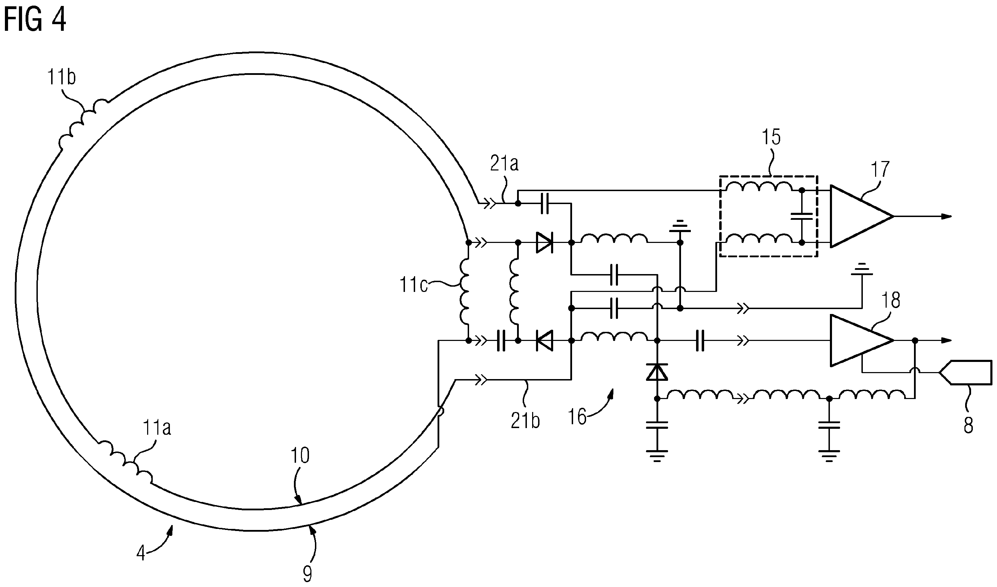

FIG 4 zeigt schematisch ein Empfangselement einer Vorrichtung in einer weiteren Ausführungsform des erfindungsgemäßen MRT-Systems 1 für einen neueren Typ von MR-Empfangsspulen, der verteilte Abstimmkapazitäten 9 anstelle von diskreten Kondensatoren verwendet, die durch parasitäre Kapazitäten zwischen Leitersegmenten wenigstens einen Leiterschleife 4 gebildet werden. Dabei werden die getrennten Leitersegmente durch die induktiven Bauelemente 11a, 11b, 11c für niedrige Frequenzen effektiv kurzgeschlossen, sodass die Leitersegmente eine Doppelschleife bilden. In FIG 4 ist auch eine Versorgungsspannung 8 für die Vorverstärkerschaltung 18 angedeutet.

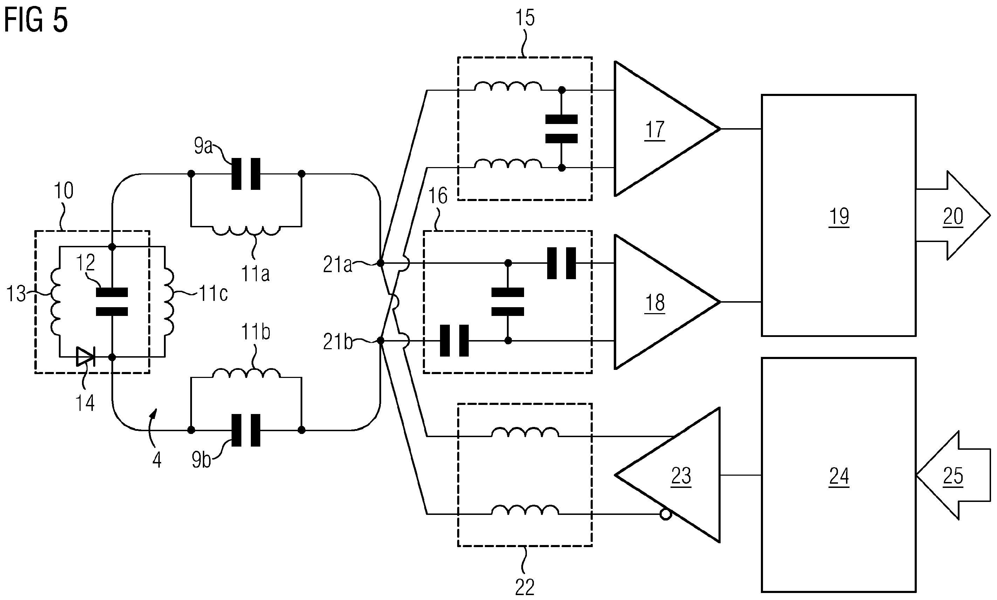

FIG 5 zeigt schematisch ein Empfangselement einer Vorrichtung in einer weiteren Ausführungsform des erfindungsgemäßen MRT-Systems 1. Das Empfangselement basiert auf dem in FIG 3 dargestellten Empfangselement.

Das Empfangselement der FIG 5 erfüllt in entsprechenden Ausführungsformen eine als lokale Abstimmung (englisch: "local shimming") bezeichnete Funktion. Dazu kann beispielsweise die Auswerteeinheit 7 oder ein sonstiger mit dem Empfangselement verbundener Computer beispielsweise einen Gleichstrom durch die wenigstens einen Leiterschleife 4 einstellen, um lokale Inhomogenitäten des statischen Magnetfeldes zu kompensieren. Der gewünschte digitale Wert des Gleichstroms wird beispielsweise über einen weiteren Datenbus 25 an einen weiteren Digital-Analog-Wandler 24 übermittelt, dessen Ausgang ein entsprechendes Signal an einen Konstantstromtreiber 23 ausgibt. Der Konstantstromtreiber 23 kann dieses Signal beispielsweise über einen weiteren Tiefpassfilter 22 den an die wenigstens eine Leiterschleife 4 übertragen.

Der weitere Tiefpassfilter 22 ist dabei insbesondere so ausgelegt, dass er den Gleichstromwert von dem Konstantstromtreiber 23 an die wenigstens eine Leiterschleife 4 überträgt und dabei die durch die pulsierenden Gradientenfelder induzierten niederfrequenten Wechselstromsignale sowie die hochfrequenten MR-Signale blockiert. Der Filterschaltkreis 15 kann dann beispielsweise als Bandpassfilter ausgeführt sein, das so konzipiert ist, dass es die durch die pulsierende Gradientenfelder induzierten Wechselstromsignale passieren lässt und die Gleichstromkomponente sowie die hochfrequenten MR-Signale unterdrückt. In analoger Weise kann auch ein Empfangselement mit verteilten Abstimmkapazitäten 9 wie in FIG 4 angepasst werden.

Die räumliche Lage von Objekten innerhalb des Bildgebungsvolumens 3 können beispielsweise durch die Verarbeitung der Signale, die von orthogonalen Spulen, die an dem Objekt angebracht sind, abhängig von den durch die pulsierenden Gradientenfelder induzierten Spannungen bestimmt werden. Eine Methode kann mit einer Anfangsschätzung beginnen und dann iterativ die Objektposition und -ausrichtung anpassen, bis bestimmte Konvergenzkriterien erfüllt sind. In einer anderen Methode wird eine Translationsmatrix in einem Pre-Trainingsschritt kalibriert, bei dem sich ein Testobjekt schrittweise bewegt, ein Bildvolumen für jeden Schritt erfasst wird gleichzeitig und die Gradientenaktivität gemessen wird. Diese Methoden können auch mit Hilfe des erfindungsgemäßen Verfahrens, des erfindungsgemäßen MRT-Systems 1 oder der Vorrichtung des erfindungsgemäßen MRT-Systems 1 kombiniert werden um die erläuterten Vorteile der Erfindung zu erzielen.

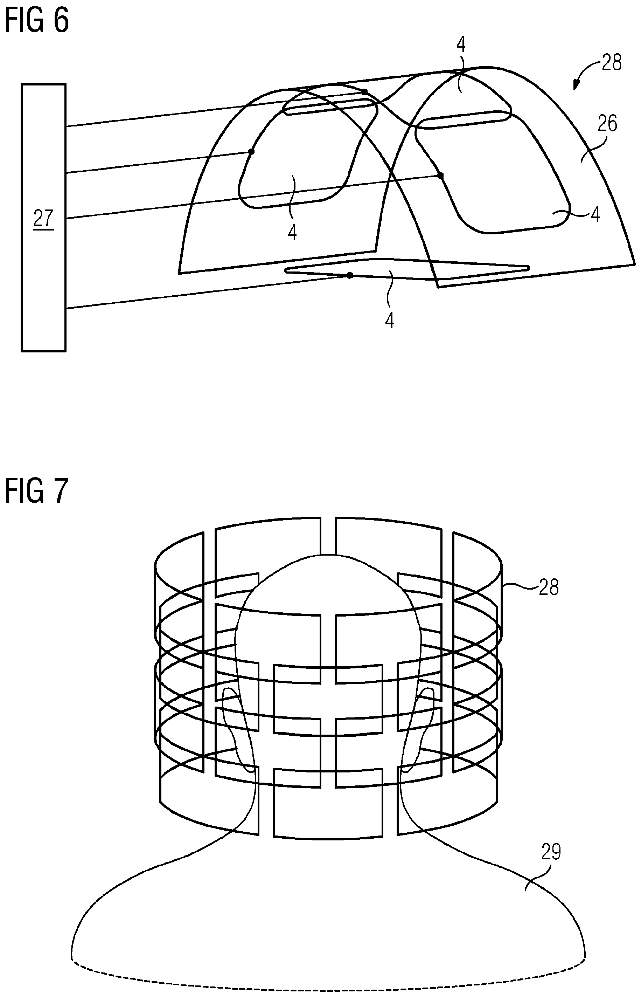

Darüber hinaus kann ein weiteres Verfahren zur Erkennung der Form und Position von lokalen flexiblen MR-Empfangsspulen 28, wie in FIG 6 bis FIG 9 schematisch dargestellt und zur Erkennung der Patientenbewegung verwendet werden. Dabei können die bereits erläuterten Vorteile der Erfindung ausgenutzt werden.

Flexible MR-Empfangsspulen 28 weisen beispielsweise eine relativ große Anzahl von Empfangselementen mit entsprechenden Leiterschleifen auf, wenn sie am Körper des Patienten 29 angebracht sind, ihre Form ändern können, wie in FIG 6 bis FIG 9 gezeigt, um der Körperkontur des Patienten 29 zu folgen, und sich eventuell auch aufgrund der Atmungsbewegung oder des Herzschlags des Patienten 29 bewegen.

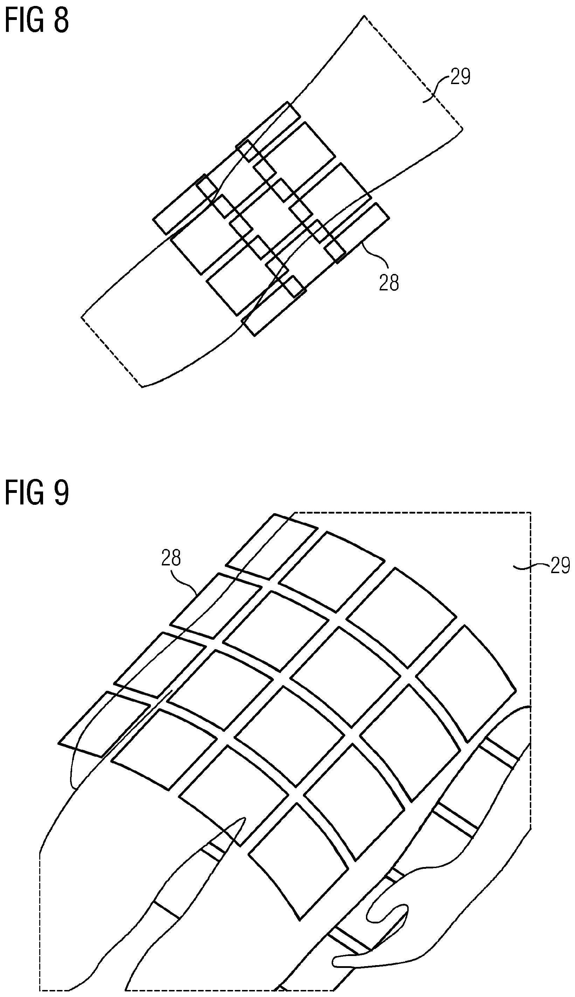

In FIG 6 ist eine flexible MR-Empfangsspule 28 mit mehreren Empfangselementen, die entsprechende Leiterschleifen 4 aufweisen, gezeigt, wobei die Empfangselemente beispielsweise in einem flexiblen Kunststoffmaterial 26 eingebettet sein können und mit einer Steuerung 27 verbunden sein können. FIG 7 zeigt schematisch eine flexible MR-Empfangsspule 28 zur Bildgebung des Kopfes des Patienten 29, FIG 8 eine flexible MR-Empfangsspule 28 zur Bildgebung des Knies des Patienten 29 und FIG 9 eine flexible MR-Empfangsspule 28 zur Bildgebung des Unterleibs des Patienten 29.

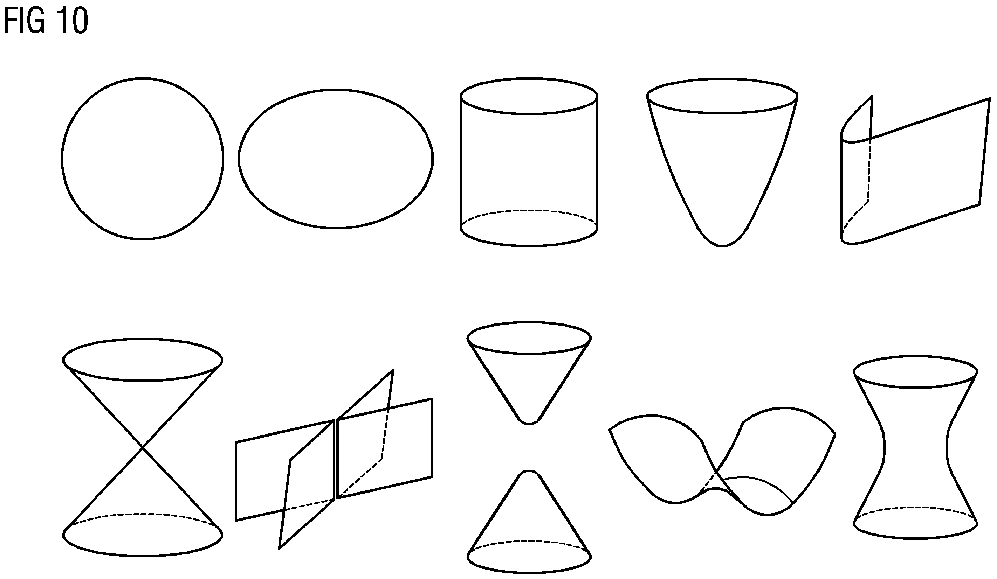

Um die Form einer flexiblen MR-Empfangsspule 28 mit hoher Genauigkeit mathematisch zu beschreiben, können mathematische Modelle für quadratische Flächen verwendet werden. Quadratische Flächen beinhalten Kugeln, Ellipsoide, Zylinder, insbesondere Kreiszylinder oder elliptische Zylinder, elliptische Paraboloide, parabolische Zylinder, Kegel, hyperbolische Zylinder, zweilagige Hyperboloide, hyperbolische Paraboloide, einlagige Hyperboloide ein- oder zweischichtige Hyperboloide, und so weiter, wie schematisch in FIG 10 in der Reihenfolge von links oben bis rechts unten dargestellt. Mathematisch gesehen ist eine quadratische Fläche der Graph einer Gleichung zweiten Grades in den drei Variablen x, y, z. Die allgemeinste Gleichungsform ist:

$$A*x^2 + B*y^2 + C*z^2 + D*x*y + E*y*z + F*x*z$$
$$+ G*x + H*y + I*z + J = 0,$$

wobei A bis J 10 Koeffizienten darstellen, die zur Anpassung die die Spulenform variiert werden können. Auf der Grundlage dieser Beobachtung können Form und Lage der flexiblen MR-Empfangsspule 28 anhand der gemessenen Spannungen bestimmt werden, die durch zeitlich veränderliche Gradientenfelder in den Empfangselementen induziert werden. Es können insbesondere folgende Schritte durchgeführt werden:

a) Initialisierung der quadratischen Fläche auf eine initiale geschätzte Form durch Zuweisung von Anfangswerten zu den Koeffizienten A bis J.
b) Initialisieren des Versatzes $(x_0, y_0)$ der Spule und des Drehwinkels der Spule in Bezug auf die x-Achse
c) Anpassung der Anordnung der Empfangselemente, also der Leiterschleifen 4 auf die quadratische Fläche.
d) Berechnen der in den Leiterschleifen 4 induzierten Spannungen unter Berücksichtigung der aktuellen Spulenform und der Gradientenstärken wie oben beschrieben.
e) Verwendung eines Gradientenabstiegsverfahrens, um die Werte der Koeffizienten A bis J, des Versatzes $(x_0, y_0)$ und des Drehwinkels so anzupassen, dass der mittlere quadratische Fehler zwischen den in Schritt d) berechneten Spannungen und den gemessenen Spannungen reduziert wird-
f) Iterative Wiederholung der Schritte c), d) und e), bis der mittlere quadratische Fehler unter einen bestimmten Schwellwert sinkt.

An dieser Stelle wird darauf hingewiesen, dass verschiedene Schritte dieses Verfahrens auch weiter optimiert werden können. Bei einer flexiblen MR-Empfangsspule 28 wie sie etwa in FIG 6 dargestellt ist, könnte beispielsweise die initiale Form so eingeschränkt werden, dass sie der Oberfläche eines Zylinders oder eines entlang der z-Achse orientierten parabolischen Zylinders entspricht. Ein parabolischer Zylinder, der entlang der x-Achse symmetrisch ist, wird mathematisch durch eine sehr einfache Gleichung beschrieben: $A*y^2 = 0$.

Für andere MR-Empfangsspulen 28 kann die Form eines oder mehrerer hyperbolischer Paraboloide besser geeignet sein. Hierfür gilt die vereinfachte Gleichung: $A*x^2 - B*y^2 + z = 0$.

Diese Art der Voroptimierung beschleunigt die Umwandlungsgeschwindigkeit des iterativen Algorithmus, indem sie die Anzahl der Koeffizienten A bis J reduziert und ein Startpunkt gesetzt, der näher an der endgültigen Lösung liegt. Die gleiche Überlegung gilt auch für den Spulenversatz und die Spulendrehung. Hier kann der numerische Bereich eingeschränkt werden, in dem sich diese Parameter ändern dürfen, und zwingt so den iterativen Algorithmus, nahe an der endgültigen Lösung zu bleiben.

Die Erfindung kann vorteilhaft auch für kabellose Spulen angewendet werden kann, die einen Analog-Digital-Wandler auf der Spule mit einer drahtlosen Übertragung digitaler kombinieren.

Die oben beschriebenen Methoden sind variabel mit bereits bekannten Methoden zur Erkennung der oder und der Patientenbewegung, etwa anhand von Hall-Sensoren, 2D- oder 3D-Videokameras oder MR-Bewegungsnavigatoren, kombiniert werden können, um die Ergebnisse weiter zu verfeinern und zu verbessern.

**Patentansprüche**

1. Verfahren zur Lagebestimmung einer Vorrichtung im Inneren eines Bildgebungsvolumens (3) eines MRT-Systems (1), wobei

   - das Bildgebungsvolumen (3) von einem Feldmagneten zum Erzeugen eines statischen Grundmagnetfelds entlang einer Längsachse (z) und von einer Gradientenspule (2) des MRT-Systems (1) umgeben ist;
   - die Vorrichtung wenigstens eine erste Leiterschleife (4) aufweist, die innerhalb einer ersten Schleifenebene verläuft;
   - mittels der Gradientenspule (2) ein magnetisches Wechselfeld in dem Bildgebungsvolumen (3) erzeugt wird;
   - mittels der wenigstens einen ersten Leiterschleife (4) wenigstens ein erster Messwert bestimmt wird, der von einer ersten Induktionsspannung abhängt, die durch eine erste Komponente des Wechselfelds senkrecht zu der Längsachse (z) in der wenigstens einen ersten Leiterschleife (4) induziert wird;
   - eine Lage der Vorrichtung im Inneren des Bildgebungsvolumens (3) abhängig von dem wenigstens einen ersten Messwert und einem vorgegebenen Magnetfeldmodell für die Gradientenspule (2) wenigstens teilweise bestimmt wird, **dadurch gekennzeichnet, dass**

   ein MR-Signal aus einem zu untersuchenden Objekt in dem Bildgebungsvolumen (3) mittels der wenigstens einen ersten Leiterschleife (4) detektiert wird und abhängig von dem MR-Signal ein MR-Bild erzeugt wird.

2. Verfahren nach Anspruch 1, wobei zum Bestimmen des wenigstens einen ersten Messwerts das MR-Signal unterdrückt wird.

3. Verfahren nach einem der vorhergehenden Ansprüche, wobei die Vorrichtung derart in dem Bildgebungsvolumen (3) positioniert wird, dass die erste Schleifenebene wenigstens näherungsweise parallel zu der Längsachse (z) ist.

4. Verfahren nach einem der vorhergehenden Ansprüche,

   - wobei die Vorrichtung wenigstens eine zweite Leiterschleife aufweist, die innerhalb einer zweiten Schleifenebene verläuft;
   - mittels der wenigstens einen zweiten Leiterschleife wenigstens ein zweiter Messwert bestimmt wird, der von einer zweiten Induktionsspannung abhängt, die durch eine zweite Komponente des Wechselfelds senkrecht zu der Längsachse (z) in der wenigstens einen zweiten Leiterschleife induziert wird; und
   - die Lage der Vorrichtung abhängig von dem wenigstens ersten Messwert, dem wenigstens einen zweiten Messwert und dem Magnetfeldmodell für die Gradientenspule (2) wenigstens teilweise bestimmt wird.

5. Verfahren nach Anspruch 4, wobei die Vorrichtung derart in dem Bildgebungsvolumen (3) positioniert wird, dass die zweite Schleifenebene wenigstens näherungsweise parallel zu der Längsachse (z) ist.

6. Verfahren nach einem der vorhergehenden Ansprüche,

   - wobei die Vorrichtung wenigstens eine dritte Leiterschleife aufweist, die innerhalb einer dritten Schleifenebene verläuft;
   - mittels der wenigstens einen dritten Leiterschleife wenigstens ein dritter Messwert bestimmt wird, der von einer dritten Induktionsspannung abhängt, die durch eine dritte Komponente des Wechselfelds senkrecht zu der Längsachse (z) in der wenigstens einen dritten Leiterschleife induziert wird; und
   - eine erste Lage der wenigstens einen ersten Leiterschleife (4) im Inneren des Bildgebungsvolumens (3) abhängig von dem wenigstens einen ersten Messwert und dem Magnetfeldmodell wenigstens teilweise bestimmt wird;
   - eine dritte Lage der wenigstens einen dritten Leiterschleife im Inneren des Bildgebungsvolumens (3) abhängig von dem wenigstens einen dritten Messwert und dem Magnetfeldmodell wenigstens teilweise bestimmt wird; und
   - eine relative Lage der wenigstens einen dritten Leiterschleife bezüglich der wenigstens einen ersten Leiterschleife (4) abhängig von der ersten Lage und der dritten Lage bestimmt wird.

7. MRT-System (1) aufweisend

   - einen Feldmagneten zum Erzeugen eines statischen Grundmagnetfelds entlang einer Längsachse (z) und eine Gradientenspule (2), wobei der Feldmagnet und die Gradientenspule (2) ein Bildgebungsvolumen (3) des MRT-Systems (1) umgeben;

- eine Vorrichtung mit wenigstens einer ersten Leiterschleife (4), die innerhalb einer ersten Schleifenebene verläuft;

- eine Steuereinheit (5), die dazu eingerichtet, die Gradientenspule (2) anzusteuern, ein magnetisches Wechselfeld in dem Bildgebungsvolumen (3) zu erzeugen;

- eine Messeinheit (6), die mit der wenigstens einen ersten Leiterschleife (4) verbunden ist und dazu eingerichtet ist, abhängig von einer ersten Induktionsspannung, die durch eine Komponente des Wechselfelds senkrecht zu der Längsachse (z) in der wenigstens einen ersten Leiterschleife (4) induziert wird, wenigstens einen ersten Messwert zu bestimmen; und

- wenigstens eine Auswerteeinheit (7), die dazu eingerichtet ist, eine Lage der Vorrichtung im Inneren eines Bildgebungsvolumens (3) abhängig von dem wenigstens einen ersten Messwert und einem vorgegebenen Magnetfeldmodell für die Gradientenspule (2) wenigstens teilweise zu bestimmen, **dadurch gekennzeichnet, dass** die Vorrichtung

- eine Abstimmkapazität (9, 9a, 9b) aufweist, die zwischen einem ersten Anschluss (21a) der wenigstens einen ersten Leiterschleife (4) und einem zweiten Anschluss (21b) der wenigstens einen ersten Leiterschleife (4) angeordnet ist; und

- ein induktives Bauelement (11a, 11b, 11c) aufweist, das elektrisch parallel zu der Abstimmkapazität (9, 9a, 9b) angeordnet ist,

wobei die erste Leiterschleife ausgebildet ist, ein MR-Signal aus dem zu untersuchenden Objekt in dem Bildgebungsvolumen mittels der wenigstens einen ersten Leiterschleife zu detektieren und die wenigstens eine Auswerteeinheit dazu eingerichtet ist, abhängig von dem MR-Signal ein MR-Bild zu erzeugen.

8. MRT-System (1) nach Anspruch 7, aufweisend eine lokale MR-Empfangsspulenanordnung (28), welche die Vorrichtung enthält, wobei die lokale MR-Empfangsspulenanordnung (28) als flexibles OberflächenspulenArray ausgestaltet ist.

9. MRT-System (1) nach Anspruch 7, aufweisend ein Gerät zur medizinischen Behandlung eines Patienten (29), wobei die wenigstens eine erste Leiterschleife (4) und das Gerät eine vorgegebene räumliche Lage zueinander aufweisen.

10. MRT-System (1) nach Anspruch 7, wobei die Messeinheit (6) einen Verstärker (17) aufweist, der mit dem ersten Anschluss (21a) und dem zweiten Anschluss (21b) verbunden ist und dazu eingerichtet ist, an einem Ausgang des Verstärkers (17), der mit der wenigstens einen Auswerteeinheit (7) verbunden ist, den wenigstens einen Messwert bereitzustellen.

11. MRT-System (1) nach Anspruch 10, wobei die Messeinheit (6) einen Filterschaltkreis (15) aufweist, der zwischen dem ersten Anschluss (21a) und einem ersten Eingang des Verstärkers (17) sowie zwischen dem zweiten Anschluss (21b) und einem zweiten Eingang des Verstärkers (17) angeordnet ist und der dazu ausgelegt ist, ein mittels der wenigstens einen Leiterschleife erfasstes MR-Signal zu unterdrücken.

## Claims

1. Method for determining the location of an apparatus inside an imaging volume (3) of an MRT system (1), wherein

- the imaging volume (3) is surrounded by a field magnet for creating a static basic magnetic field along a longitudinal axis (z) and by a gradient coil (2) of the MRT system (1);
- the apparatus has at least one first conductor loop (4), which runs within a first loop plane;
- by means of the gradient coil (2) a magnetic alternating field is created in the imaging volume (3);
- by means of the at least one first conductor loop (4) at least one first measured value is determined, which depends on a first induction voltage, which is induced by a first component of the alternating field at right angles to the longitudinal axis (z) in the at least one first conductor loop (4);
- a location of the apparatus inside the imaging volume (3) is determined at least partly as a function of at least one first measured value and a predetermined magnetic field model for the gradient coil (2),

**characterised in that**
an MR signal from an object to be examined in the imaging volume (3) is detected by means of the at least one first conductor loop (4) and an MR image is created as a function of the MR signal.

2. Method according to claim 1 wherein, to determine the at least one first measured value, the MR signal is suppressed.

3. Method according to one of the preceding claims, wherein the apparatus is positioned in the imaging volume (3) in such a way that the first loop plane is at least approximately parallel to the longitudinal axis (z).

4. Method according to one of the preceding claims,

   - wherein the apparatus has at least one second conductor loop that runs within a second loop plane;
   - by means of the at least one second conductor loop at least one second measured value is determined, which depends on a second induction voltage that is induced by a second component of the alternating field at right angles to the longitudinal axis (z) in the at least one second conductor loop; and
   - the location of the apparatus is determined at least partly as a function of at least first measured value, the at least one second measured value and the magnetic field model for the gradient coil (2).

5. Method according to claim 4, wherein the apparatus is positioned in the imaging volume (3) in such a way that the second loop plane is at least approximately parallel to the longitudinal axis (z).

6. Method according to one of the preceding claims,

   - wherein the apparatus has at least one third conductor loop, which runs within a third loop plane;
   - by means of the at least one third conductor loop at least one third measured value is determined, which depends on a third induction voltage that is induced by a third component of the alternating field at right angles to the longitudinal axis (z) in the at least one third conductor loop; and
   - a first location of the at least one first conductor loop (4) inside the imaging volume (3) is determined at least partly as a function of the at least one first measured value and the magnetic field model;
   - a third location of the at least one third conductor loop inside the imaging volume (3) is determined at least partly as a function of the at least one third measured value and the magnetic field model; and
   - a relative location of the at least one third conductor loop with regard to the at least one first conductor loop (4) is determined as a function of the first location and the third location.

7. MRT system (1) having

   - a field magnet for creating a static basic magnetic field along a longitudinal axis (z) and a gradient coil (2), wherein the field magnet and the gradient coil (2) surround an imaging volume (3) of the MRT system (1);
   - an apparatus with at least one first conductor loop (4), which runs within a first loop plane;
   - a control unit (5), which is configured to activate the gradient coil (2) to create a magnetic alternating field in the imaging volume (3);
   - a measurement unit (6), which is connected to the at least one first conductor loop (4) and is configured, as a function of a first induction voltage that is induced by a component of the alternating field at right angles to the longitudinal axis (z) in the at least one first conductor loop (4), to determine at least one first measured value; and
   - at least one evaluation unit (7), which is configured to determine a location of the apparatus inside an imaging volume (3) at least partly as a function of at least one first measured value and a predetermined magnetic field model for the gradient coil (2),

   **characterised in that**

   the apparatus

   - has a tuning capacitance (9, 9a, 9b), which is arranged between a first terminal (21a) of the at least one first conductor loop (4) and a second terminal (21b) of the at least one first conductor loop (4); and
   - has an inductive component (11a, 11b, 11c), which is arranged electrically in parallel to the tuning capacitance (9, 9a, 9b),

   wherein the first conductor loop is designed to detect an MR signal from the object to be examined in the imaging volume by means of the at least one first conductor loop and the evaluation unit is configured, depending on the MR signal, to create an MR image.

8. MRT system (1) according to claim 7, having a local MR receive coil arrangement (28), which contains the apparatus, wherein the local MR receive coil arrangement (28) is embodied as a flexible surface coil array.

9. MRT system (1) as claimed in claim 7, having a device for medical treatment of a patient (29), wherein the at least one first conductor loop (4) and the device have a predetermined spatial location in relation to one another.

10. MRT system (1) according to claim 7, wherein the measurement unit (6) has an amplifier (17), which is connected to the first terminal (21a) and the second terminal (21b) and is configured to provide the at least one measured value at an output of the amplifier (17), which is connected to the at least one evaluation unit (7).

11. MRT system (1) according to in claim 10, wherein the measurement unit (6) has a filter circuit (15), which is arranged between the first terminal (21a) and a first input of the amplifier (17) as well as between the second terminal (21b) and a second input of the amplifier (17) and which is designed to suppress an MR signal acquired by means of the at least one conductor loop.

**Revendications**

1. Procédé de la détermination de la position d'un dispositif à l'intérieur d'un volume (3) d'imagerie d'un système (1) d'IRM, dans lequel

- le volume (3) d'imagerie est entouré d'un champ magnétique pour la production d'un champ magnétique de base statique le long d'un axe (z) longitudinal et d'une bobine (2) de gradient du système (1) d'IRM ;
- le dispositif a au moins une première boucle (4) conductrice, qui s'étend dans un premier plan de boucle ;
- on produit, au moyen de la bobine (2) de gradient, un champ magnétique alternatif dans le volume (3) d'imagerie ;
- au moyen de la au moins une première boucle (4) conductrice, on détermine au moins une première valeur de mesure, qui dépend d'une première tension d'induction, que l'on induit dans la au moins une première boucle (4) conductrice par une première composante du champ alternatif perpendiculaire à l'axe (z) longitudinal ;
- on détermine au moins en partie une position du dispositif à l'intérieur du volume (3) d'imagerie, en fonction de la au moins une première valeur de mesure et d'un modèle de champ magnétique donné à l'avance pour la bobine (2) de gradient,

**caractérisé en ce que**
l'on détecte, au moyen de la au moins une première boucle (4) conductrice, un signal RM provenant d'un objet à étudier dans le volume (3) d'imagerie et on produit une image RM en fonction du signal RM.

2. Procédé suivant la revendication 1, dans lequel, pour la détermination de la au moins une première valeur de mesure, on supprime le signal RM.

3. Procédé suivant l'une des revendications précédentes, dans lequel on met le dispositif en position dans le volume (3) d'imagerie, de manière à ce que le premier plan de boucle soit au moins à peu près parallèle à l'axe (z) longitudinal.

4. Procédé suivant l'une des revendications précédentes,

- dans lequel le dispositif a au moins une deuxième boucle conductrice, qui s'étend dans un deuxième plan de boucle ;
- au moyen de la au moins une deuxième boucle conductrice, on détermine au moins une deuxième valeur de mesure, qui dépend d'une deuxième tension d'induction, qui est induite par une deuxième composante du champ alternatif perpendiculaire à l'axe (z) longitudinal ; et
- on détermine au moins en partie la position du dispositif en fonction de la au moins une première valeur de mesure, de la au moins une deuxième valeur de mesure et du modèle de champ magnétique pour la bobine (2) de gradient.

5. Procédé suivant la revendication 4, dans lequel on met le dispositif en position dans le volume (3) d'imagerie, de manière à ce que le deuxième plan de boucle soit au moins à peu près parallèle à l'axe (z) longitudinal.

6. Procédé suivant l'une des revendications précédentes,

- dans lequel le dispositif a au moins une troisième boucle conductrice, qui s'étend dans un troisième plan de boucle ;
- au moyen de la au moins une troisième boucle conductrice, on détermine au moins une troisième valeur de mesure, qui dépend d'une troisième tension d'induction, qui est induite dans la au moins une troisième bobine conductrice par une troisième composante du champ alternatif perpendiculaire à l'axe (z) longitudinal ; et
- on détermine au moins en partie une première position de la au moins une première boucle (4) conductrice à l'intérieur du volume (3) d'imagerie, en fonction de la au moins une première valeur de mesure et du modèle de champ magnétique ;
- on détermine au moins en partie une troisième position de la au moins une troisième boucle conductrice à l'intérieur du volume (3) d'imagerie, en fonction de la au moins une troisième valeur de mesure et du modèle de champ magnétique ; et
- on détermine, en fonction de la première position et de la troisième position, une position relative de la au moins une troisième boucle conductrice par rapport à la au moins une première boucle (4) conductrice.

7. Système (1) d'IRM comportant

- un aimant de champ pour la production d'un champ magnétique de base statique le long d'un axe (z) longitudinal et une bobine (2) de gradient, dans lequel le champ magnétique et la bobine (2) de gradient entourent un volume (3) d'imagerie du système (1) d'IRM.
- un dispositif ayant au moins une première boucle (4) conductrice, qui s'étend à l'intérieur d'un premier plan de boucle ;
- une unité (5) de commande, qui est agencée pour commander la bobine (2) de gradient, afin de produire un champ magnétique alternatif dans le volume (3) d'imagerie ;
- une unité (6) de mesure, qui est connectée à la au moins une première boucle (4) conductrice et qui est agencée pour, en fonction d'une première tension d'induction, que l'on induit dans la au moins une première boucle (4) conductrice par une composante du champ alternatif perpendiculaire à l'axe (z) longitudinal, déterminer au moins une première valeur de mesure ; et
- au moins une unité (7) d'évaluation, qui est agencée pour déterminer au moins en partie une position du dispositif à l'intérieur d'un volume (3) d'imagerie, en fonction de la au moins une première valeur de mesure et d'un modèle de champ magnétique donné à l'avance pour la bobine (2) de gradient,

**caractérisé en ce que**
le dispositif

- a une capacité (9, 9a, 9b) d'accord, qui est montée entre une première borne (21a) de la au moins une première boucle (4) conductrice et une deuxième borne (21b) de la au moins une première boucle (4) conductrice ; et
- un composant (11a, 11b, 11c) inducteur, qui est monté électriquement en parallèle avec la capacité (9, 9a, 9b) d'accord, dans lequel la première boucle conductrice est constituée pour détecter, au moyen de la au moins une première boucle conductrice, un signal RM provenant de l'objet à étudier et la au moins une unité d'évaluation est agencée pour produire une image RM en fonction du signal RM.

8. Système (1) d'IRM suivant la revendication 7, comportant un agencement (28) de bobines de réception RM local, qui contient le dispositif, dans lequel l'agencement (28) de bobines de réception RM local est conformé sous la forme d'un réseau souple de bobines de surface.

9. Système (1) d'IRM suivant la revendication 7, comportant un appareil de traitement médical d'un patient (29), dans lequel la au moins une première boucle (4) conductrice et l'appareil ont une position dans l'espace donnée à l'avance l'un par rapport à l'autre.

10. Système (1) d'IRM suivant la revendication 7, dans lequel l'unité (6) de mesure a un amplificateur (17), qui est connecté à la première borne (21a) et à la deuxième borne (21b) et qui est agencé pour donner la au moins une valeur de mesure à une sortie de l'amplificateur (17), qui est connecté à la au moins une unité (7) d'évaluation.

11. Système (1) d'IRM suivant la revendication 10, dans lequel l'unité (6) de mesure a un circuit (15) de filtrage, qui est monté entre la première borne (21a) et une première entrée de l'amplificateur (17), ainsi qu'entre la deuxième borne (21b) et une deuxième entrée de l'amplificateur (17) et qui est agencée pour supprimer un signal RM détecté au moyen de la au moins une boucle conductrice.

## FIG 1

## FIG 2

FIG 3

EP 4 224 189 B1

FIG 4

EP 4 224 189 B1

FIG 5

EP 4 224 189 B1

# FIG 6

# FIG 7

FIG 8

FIG 9

FIG 10

EP 4 224 189 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0013586 A1
- US 2021212588 A1
- US 2003184297 A1
- US 2007145978 A1

**In der Beschreibung aufgeführte Nicht-Patentliteratur**

- **JAMES A SMITH et al.** A Novel Position and Orientation Sensor for MRI. *PROCEEDINGS OF THE INTERNATIONAL SOCIETY FOR MAGNETIC RESONANCE IN MEDICINE, ISMRM, 25TH ANNUAL MEETING AND EXHIBITION*, 07 April 2017 (3930), 3930